# EUROPEAN PATENT APPLICATION

(11) **EP 4 438 602 A1**
(43) Date of publication of application: **02.10.2024**
(21) Application number: 23740287.0
(22) Date of filing: 12.01.2023
(51) Int. Cl.: C07D 451/02, A61K 31/506, A61P 25/00, A61P 43/00

(54) **CRYSTAL OF SUBSTITUTED PIPERIDINE COMPOUND, SALTS OF SUBSTITUTED PIPERIDINE COMPOUND, AND CRYSTALS THEREOF**

(30) Priority: 14.01.2022 JP 2022004608
(71) Applicant: Eisai R&D Management Co., Ltd., Tokyo 112-8088 (JP)
(72) Inventor: KUSHIDA Ikuo, Tsukuba-shi, Ibaraki 300-2635 (JP); ITO Yoko, Tsukuba-shi, Ibaraki 300-2635 (JP); YASUI So, Tsukuba-shi, Ibaraki 300-2635 (JP); FUKUYAMA Takashi, Kamisu-shi, Ibaraki 314-0255 (JP); SATO Nobuaki, Tsukuba-shi, Ibaraki 300-2635 (JP); ASABA Taro, Tsukuba-shi, Ibaraki 300-2635 (JP)
(74) Representative: Hoffmann Eitle
(86) International application number: PCT/JP2023/000561
(87) International publication number: WO 2023/136279

(57) **Abstract**

Crystals of compounds represented by formula (I) having potential as drug substances for pharmaceuticals, as well as salts of compounds represented by formula (I) and their crystals.

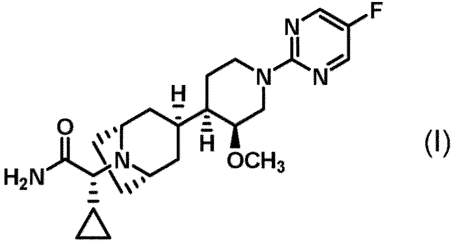

## Description

### Technical Field

The present invention relates to crystal of substituted piperidine compound, as well as salts of substituted piperidine compound and their crystals, having orexin type 2 receptor activating activity.

### Background Art

Orexin-A (OX-A) and orexin-B (OX-B), two types of intracerebral neuropeptides produced specifically by specific neurons localized in the outer field of the cerebral hypothalamus, were discovered as endogenous ligands of orexin receptors (PTLs 1-4), which are G protein-coupled receptors present mainly in the cerebrum (see PTL 5 and NPL 1). Orexin receptors are known to have two subtypes, OX₁ receptor (OX1R) as subtype 1 and OX₂ receptor (OX2R) as subtype 2. Orexins have been found to promote rat feeding behavior (NPL 1).

An OX2R gene mutation has been reported to be one cause of canine narcolepsy (NPL 2), and orexin-knockout mice exhibit narcolepsy-like symptoms that are highly similar to human or canine narcolepsy (NPL 3). Research using transgenic mice with modified orexin neurons, and double transgenic mice obtained by cross-breeding these mice with orexin-overexpressing transgenic mice, has shown that narcolepsy-like symptoms exhibited by modification of orexin neurons are eliminated by persistent expression of orexin (NPL 4). Similarly, it has been found that intracerebroventricular administration of OX-A to transgenic mice with modified orexin neurons inhibits cataplexy (affective cataplexy)-like stopping action, and improves symptoms of narcolepsy by increasing alertness, for example (NPL 4). Research in OX2R knockout mice also suggests that OX2R is important for maintaining alertness (NPL 5). It has also been suggested that loss of orexin nerves is a cause of daytime sleepiness in Parkinson's disease patients (NPL 6). Furthermore it has been suggested that plasma OX-A concentration levels are low in sleep apnea syndrome patients (NPL 7). On this basis, it was suggested that OX2R agonists can serve as narcolepsy treatments or therapeutic agents for other sleep disorders that exhibit hypersomnia (NPL 8).

It is therefore believed that compounds with OX2R-agonist activity can be utilized as therapeutic agents for narcolepsy, idiopathic hypersomnia, hypersomnia, sleep apnea syndrome, states of impaired consciousness such as coma, narcolepsy syndrome accompanied by narcolepsy-like symptoms, and hypersomnia syndrome accompanied by daytime hypersomnia (for example, Parkinson's disease, Guillain-Barré syndrome and Kleine Levin syndrome).

TAK-925, a compound with OX2R-agonist activity, has entered phase I trials for healthy persons and narcolepsy patients (intravenous administration).

### Citation List

### Patent Literature

[PTL 1] International Patent Publication No. WO1996/34877
[PTL 2] Japanese Unexamined Patent Publication HEI No. 10-327888
[PTL 3] Japanese Unexamined Patent Publication HEI No. 10-327889
[PTL 4] Japanese Unexamined Patent Publication HEI No. 11-178588
[PTL 5] Japanese Unexamined Patent Publication HEI No. 10-229887

### Non-Patent Literature

[NPL 1] Sakurai T.et al, Cell, 1998,92,573-585
[NPL 2] Lin L. et al, Cell, 1999,98,365-376
[NPL 3] Chemelli R.M.et al, Cell, 1999,98,437-451
[NPL 4] Mieda M.et al, Proc. Natl. Acad. Sci. USA, 2004,101,4649-4654
[NPL 5] Willie J.T. et al, Neuron, 2003,38,715-730
[NPL 6] Thannickal T.C.et al, Brain 2007,130,1586-1595
[NPL 7] Busquets X.et al, Respiration, 2004,71,575-579
[NPL 8] Mieda M.et al, CNS Drugs, 2013,27,83-90

### Summary of Invention

### Technical Problem

Compounds represented by the following formula (I) ((2R)-2-cyclopropyl-2- { (1R,3 S, 5 S)-3 -[(3 S,4R)-1 -(5-fluoropyrimidin-2-yl)-3 - methoxypiperidin-4-yl]-8-azabicyclo[3.2. 1]octan-8-yl}acetamide, hereunder also collectively referred to as "compound (I)") have orexin type 2 receptor agonist activity, and have the potential for use as therapeutic agents for narcolepsy, for example.

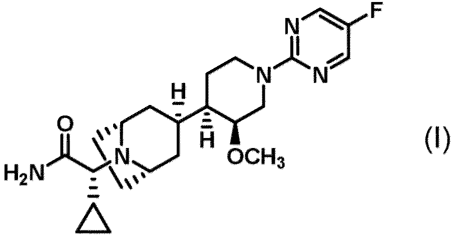

The physical properties of compounds and their salts used in pharmaceuticals, as well as their crystals, generally have major effects on the bioavailability of the drugs, on bulk drug purity, and on formulation prescription. It is therefore an object of the invention to provide crystals of compound (I), as well as salts of compound (I) and their crystals, which can be used as drug substances for pharmaceuticals.

### Solution to Problem

The present inventors have completed this invention as a result of much research on compound (I) in light of the circumstances described above, and upon discovering crystals of compound (I), and salts of compound (I) and their crystals.

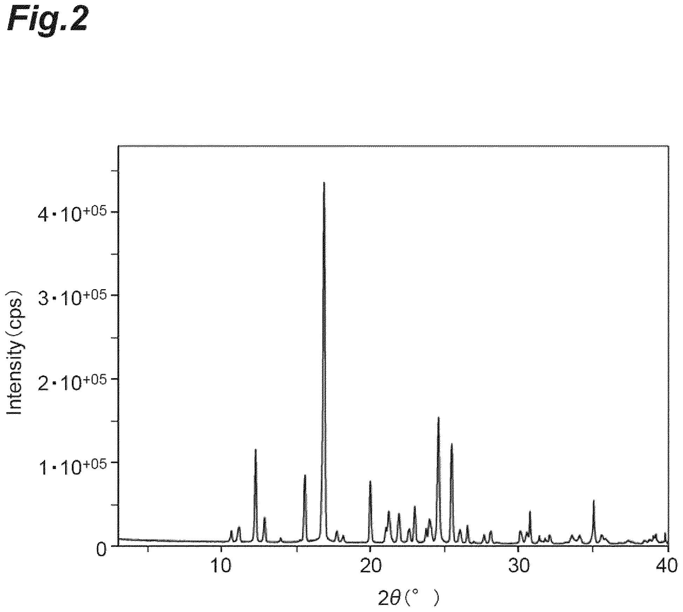

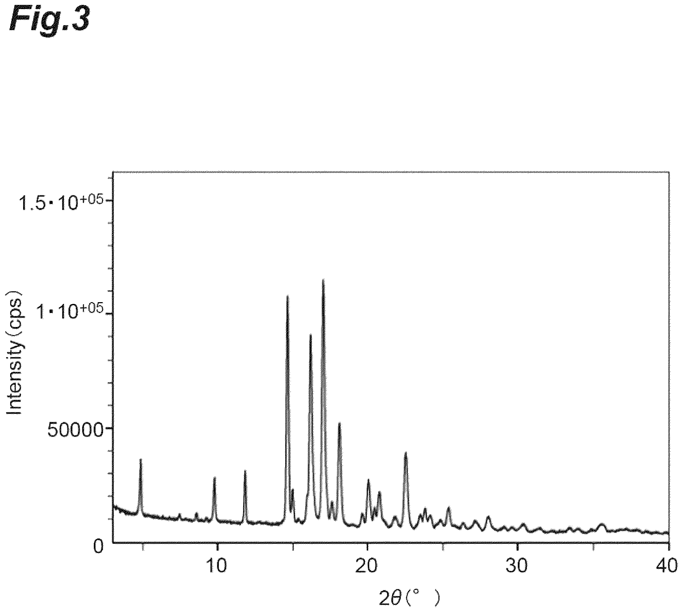

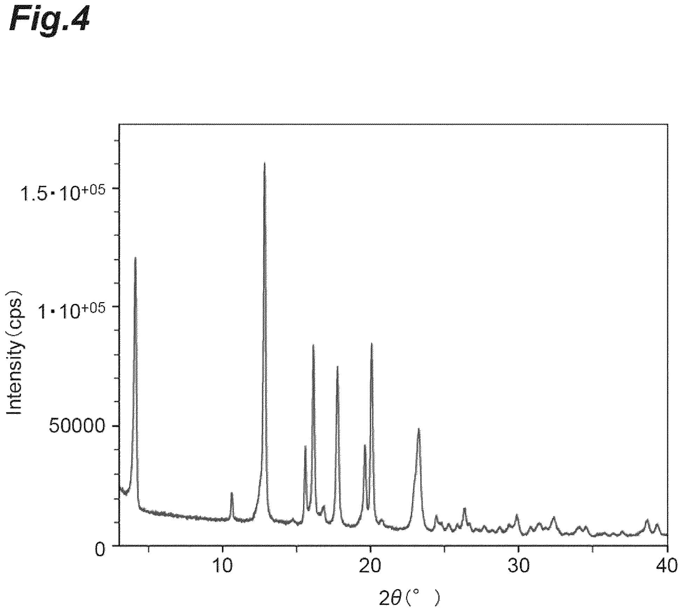

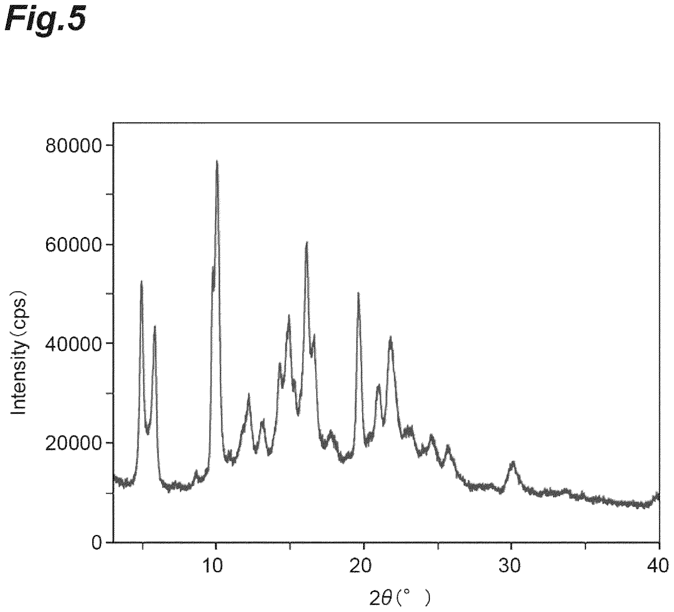

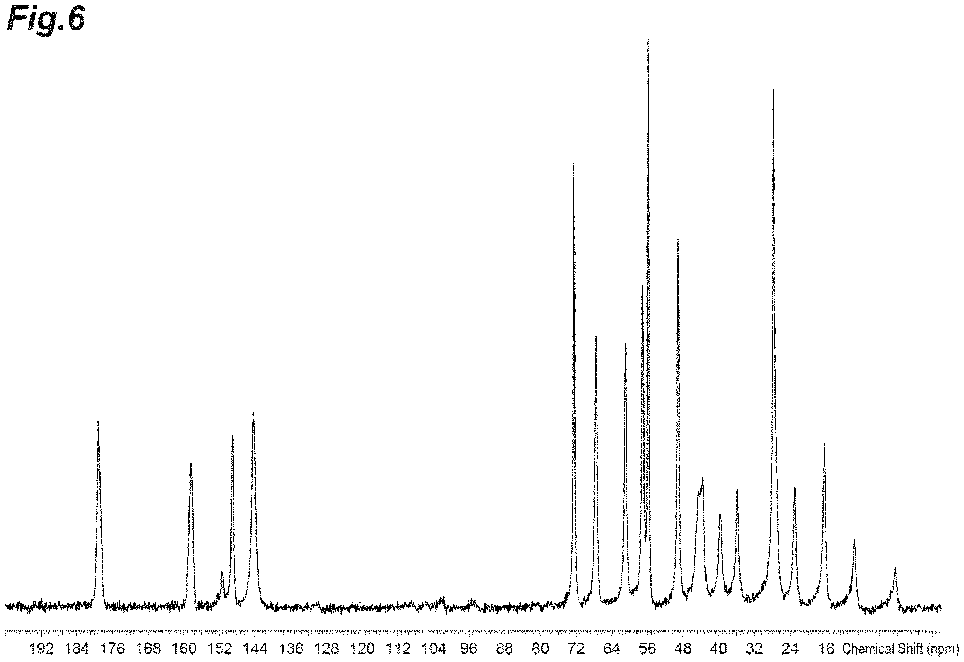

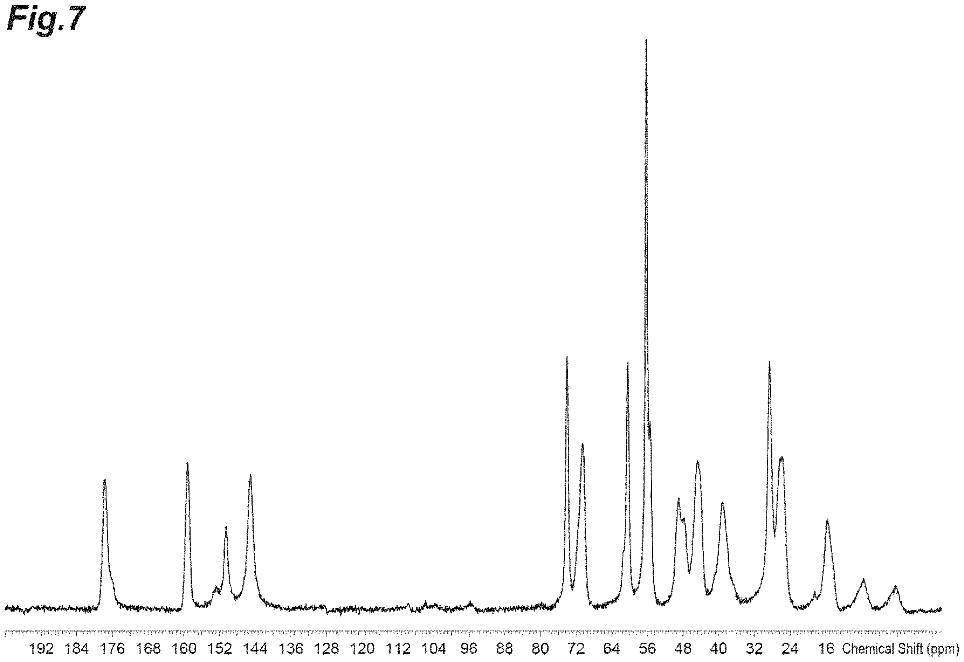

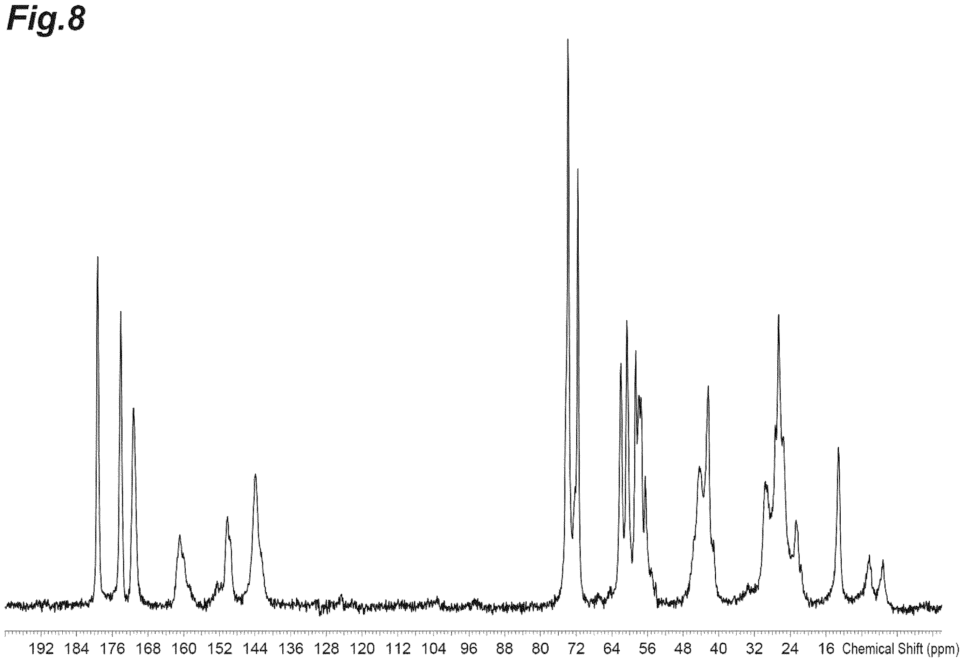

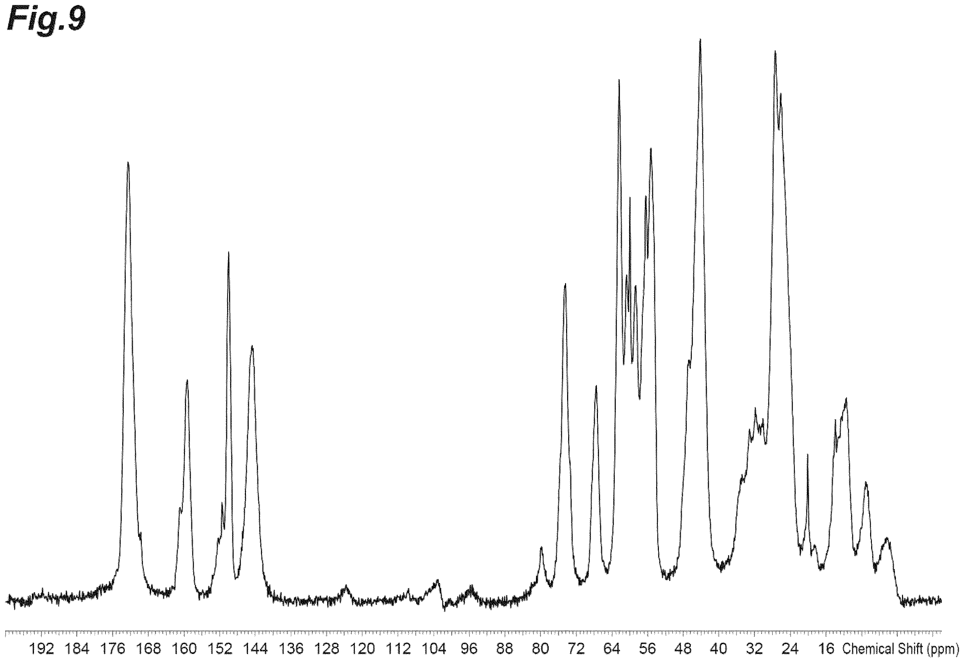

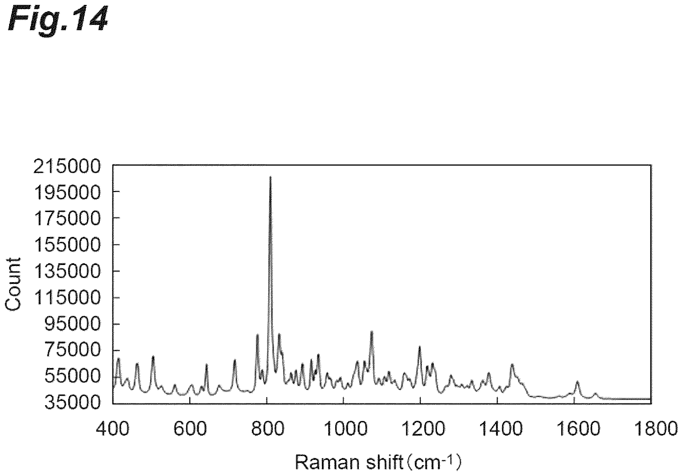

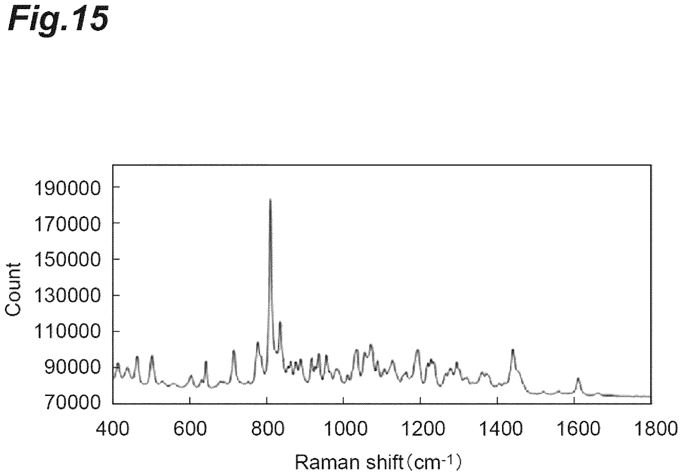

Specifically, the invention relates to the following [1] to [52].
[1] Mono D-tartrate or hemioxalate of (2R)-2-cyclopropyl-2-{(1R,3S,5S)-3-[(3 S,4R)-1-(5-fluoropyrimidin-2-yl)-3-methoxypiperidin-4-yl]-8-azabicyclo[3.2.1]octan-8-yl}acetamide represented by the following formula (I): 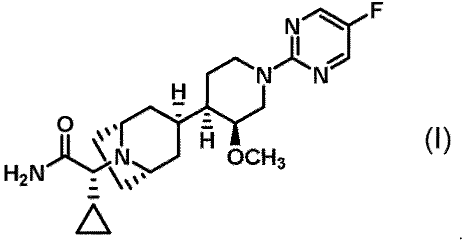
[2] A crystal of mono D-tartrate or hemioxalate of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide.
[3] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl} acetamide, having one or more diffraction peaks selected from the group consisting of diffraction angles (2θ ±0.2°) of 12.2°, 12.8°, 15.5°, 21.2° and 24.6° in a powder X-ray diffraction.
[4] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a diffraction peak at a diffraction angle (2θ ±0.2°) of 24.6° in a powder X-ray diffraction.
[5] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having diffraction peaks at diffraction angles (2θ ±0.2°) of 12.2°, 15.5° and 24.6° in a powder X-ray diffraction.
[5.1] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having diffraction peaks at diffraction angles (2θ ±0.2°) of 12.2°, 12.8°, 15.5°, 21.2° and 24.6° in a powder X-ray diffraction.
[5.2] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having diffraction peaks at diffraction angles (2θ ±0.2°) of 10.6°, 11.1°, 12.2°, 12.8°, 15.5°, 21.2°, 23.0°, 24.6°, 26.0° and 35.0° in a powder X-ray diffraction.
[6] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a powder X-ray diffraction pattern substantially the same as the powder X-ray diffraction pattern shown in Fig. 2.
[7] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a peak at a chemical shift (δ ±0.5 ppm) of 72.5 ppm in a solid state ¹³C NMR spectrum.
[8] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having peaks at chemical shifts (δ ±0.5 ppm) of 49.2 ppm, 67.6 ppm and 72.5 ppm in a solid state ¹³C NMR spectrum.
[8.1] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having peaks at chemical shifts (δ ±0.5 ppm) of 16.4 ppm, 49.2 ppm, 67.6 ppm, 72.5 ppm and 179.1 ppm in a solid state ¹³C NMR spectrum.
[8.2] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having peaks at chemical shifts (δ ±0.5 ppm) of 16.4 ppm, 27.7 ppm, 49.2 ppm, 55.9 ppm, 60.9 ppm, 67.6 ppm, 72.5 ppm, 144.4 ppm and 179.1 ppm in a solid state ¹³C NMR spectrum.
[9] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a solid state ¹³C NMR spectrum substantially the same as the solid state ¹³C NMR spectrum shown in Fig. 6.
[10] AForm α crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide, having a Raman shift peak (±2 cm⁻¹) at 1199.4 cm⁻¹ in Raman spectrophotometry.
[11] AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having Raman shift peaks (±2 cm⁻¹) at 842.1 cm⁻¹, 895.0 cm⁻¹ and 1199.4 cm⁻¹ in Raman spectrophotometry.
[11.1] AForm α crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide, having Raman shift peaks (±2 cm⁻¹) at 811.6 cm⁻¹, 842.1 cm⁻¹, 895.0 cm⁻¹, 1074.8 cm⁻¹ and 1199.4 cm⁻¹ in Raman spectrophotometry.
[11.2] AForm α crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide, having Raman shift peaks (±2 cm⁻¹) at 508.1 cm⁻¹, 778.4 cm⁻¹, 811.6 cm⁻¹, 834.5 cm⁻¹, 842.1 cm⁻¹, 895.0 cm⁻¹, 934.9 cm⁻¹, 1074.8 cm⁻¹, 1094.2 cm⁻¹ and 1199.4 cm⁻¹ in Raman spectrophotometry.
[12] AForm α crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide, having a Raman spectrum substantially the same as the Raman spectrum shown in Fig. 14.
[13] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl} acetamide, having one or more diffraction peaks selected from the group consisting of diffraction angles (2θ ±0.2°) of 4.9°, 9.8°, 11.8°, 14.6° and 16.1° in a powder X-ray diffraction.
[14] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a diffraction peak at a diffraction angle (2θ ±0.2°) of 14.6° in a powder X-ray diffraction.
[15] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having diffraction peaks at diffraction angles (2θ ±0.2°) of 11.8°, 14.6° and 16.1° in a powder X-ray diffraction.
[15.1] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having diffraction peaks at diffraction angles (2θ ±0.2°) of 4.9°, 9.8°, 11.8°, 14.6° and 16.1° in a powder X-ray diffraction.
[15.2] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having diffraction peaks at diffraction angles (2θ ±0.2°) of 4.9°, 9.8°, 11.8°, 14.6°, 15.0°, 16.1°, 19.6°, 20.4°, 20.7° and 23.5° in a powder X-ray diffraction.
[16] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a powder X-ray diffraction pattern substantially the same as the powder X-ray diffraction pattern shown in Fig. 3.
[17] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a peak at a chemical shift (δ ±0.5 ppm) of 74.1 ppm in a solid state ¹³C NMR spectrum.
[18] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having peaks at chemical shifts (δ ±0.5 ppm) of 74.1 ppm, 159.2 ppm and 177.7 ppm in a solid state ¹³C NMR spectrum.
[18.1] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having peaks at chemical shifts (δ ±0.5 ppm) of 39.1 ppm, 70.6 ppm, 74.1 ppm, 159.2 ppm and 177.7 ppm in a solid state ¹³C NMR spectrum.
[18.2] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having peaks at chemical shifts (δ ±0.5 ppm) of 28.6 ppm, 39.1 ppm, 56.3 ppm, 60.4 ppm, 70.6 ppm, 74.1 ppm, 145.0 ppm, 150.5 ppm, 159.2 ppm and 177.7 ppm in a solid state ¹³C NMR spectrum.
[19] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a solid state ¹³C NMR spectrum substantially the same as the solid state ¹³C NMR spectrum shown in Fig. 7.
[20] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a Raman shift peak (±2 cm⁻¹) at 1089.4 cm⁻¹ in Raman spectrophotometry.
[21] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having Raman shift peaks (±2 cm⁻¹) at 889.9 cm⁻¹, 1089.4 cm⁻¹ and 1194.6 cm⁻¹ in Raman spectrophotometry.
[21.1] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having Raman shift peaks (±2 cm⁻¹) at 811.6 cm⁻¹, 837.0 cm⁻¹, 889.9 cm⁻¹, 1089.4 cm⁻¹ and 1194.6 cm⁻¹ in Raman spectrophotometry.
[21.2] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having Raman shift peaks (±2 cm⁻¹) at 716.6 cm⁻¹, 778.4 cm⁻¹, 811.6 cm⁻¹, 837.0 cm⁻¹, 889.9 cm⁻¹, 1035.9 cm⁻¹, 1072.4 cm⁻¹, 1089.4 cm⁻¹, 1194.6 cm⁻¹ and 1442.2 cm⁻¹ in Raman spectrophotometry.
[22] AForm β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide, having a Raman spectrum substantially the same as the Raman spectrum shown in Fig. 15.
[23] A crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide mono D-tartrate, having one or more diffraction peaks selected from the group consisting of diffraction angles (2θ ±0.2°) of 4.1°, 12.8°, 16.1°, 20.0° and 23.3° in a powder X-ray diffraction.
[24] A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate, having a diffraction peak at a diffraction angle (2θ ±0.2°) of 4.1° in a powder X-ray diffraction.
[25] A crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide mono D-tartrate, having diffraction peaks at diffraction angles (2θ ±0.2°) of 4.1°, 12.8° and 23.3° in a powder X-ray diffraction.
[25.1] Acrystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate, having diffraction peaks at diffraction angles (2θ ±0.2°) of 4.1°, 12.8°, 16.1°, 20.0° and 23.3° in a powder X-ray diffraction.
[25.2] Acrystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide mono D-tartrate, having diffraction peaks at diffraction angles (2θ ±0.2°) of 4.1°, 10.6°, 12.8°, 15.6°, 16.1°, 17.7°, 19.6°, 20.0°, 23.3° and 32.3° in a powder X-ray diffraction.
[26] A crystal of (2R)-2-cyclopropyl-2-{(1R,3 S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate, having a powder X-ray diffraction pattern substantially the same as the powder X-ray diffraction pattern shown in Fig. 4.
[27] A crystal of (2R)-2-cyclopropyl-2-{(1R,3 S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate, having a peak at a chemical shift (δ ±0.5 ppm) of 73.8 ppm in a solid state ¹³C NMR spectrum.
[28] A crystal of (2R)-2-cyclopropyl-2-{(1R,3 S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate, having peaks at chemical shifts (δ ±0.5 ppm) of 71.6 ppm, 73.8 ppm and 179.3 ppm in a solid state ¹³C NMR spectrum.
[28.1] Acrystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide mono D-tartrate, having peaks at chemical shifts (δ ±0.5 ppm) of 26.6 ppm, 71.6 ppm, 73.8 ppm, 174.1 ppm and 179.3 ppm in a solid state ¹³C NMR spectrum.
[28.2] Acrystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide mono D-tartrate, having peaks at chemical shifts (δ ±0.5 ppm) of 13.2 ppm, 26.6 ppm, 42.3 ppm, 60.6 ppm, 62.0 ppm, 71.6 ppm, 73.8 ppm, 171.2 ppm, 174.1 ppm and 179.3 ppm in a solid state ¹³C NMR spectrum.
[29] A crystal of (2R)-2-cyclopropyl-2-{(1R,3 S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate, having a solid state ¹³C NMR spectrum substantially the same as the solid state ¹³C NMR spectrum shown in Fig. 8.
[30] A crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide hemioxalate, having one or more diffraction peaks selected from the group consisting of diffraction angles (2θ ±0.2°) of 5.0°, 5.8°, 10.0°, 13.2° and 14.3° in a powder X-ray diffraction.
[31] A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate, having a diffraction peak at a diffraction angle (2θ ±0.2°) of 10.0° in a powder X-ray diffraction.
[32] A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate, having diffraction peaks at diffraction angles (2θ ±0.2°) of 5.8°, 10.0° and 14.3° in a powder X-ray diffraction.
[32.1] Acrystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide hemioxalate, having diffraction peaks at diffraction angles (2θ ±0.2°) of 5.0°, 5.8°, 10.0°, 13.2° and 14.3° in a powder X-ray diffraction.
[32.2] Acrystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide hemioxalate, having diffraction peaks at diffraction angles (2θ ±0.2°) of 5.0°, 5.8°, 7.2°, 8.6°, 9.7°, 10.0°, 12.3°, 13.2°, 14.3° and 14.9° in a powder X-ray diffraction.
[33] A crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide hemioxalate, having a powder X-ray diffraction pattern substantially the same as the powder X-ray diffraction pattern shown in Fig. 5.
[34] A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate, having a peak at a chemical shift (δ ±0.5 ppm) of 44.1 ppm in a solid state ¹³C NMR spectrum.
[35] A crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide hemioxalate, having peaks at chemical shifts (δ ±0.5 ppm) of 27.3 ppm, 44.1 ppm and 62.4 ppm in a solid state ¹³C NMR spectrum.
[35.1] A crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide hemioxalate, having peaks at chemical shifts (δ ±0.5 ppm) of 27.3 ppm, 44.1 ppm, 55.2 ppm, 62.4 ppm and 172.5 ppm in a solid state ¹³C NMR spectrum.
[35.2] A crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide hemioxalate, having peaks at chemical shifts (δ ±0.5 ppm) of 27.3 ppm, 44.1 ppm, 55.2 ppm, 62.4 ppm, 67.5 ppm, 74.4 ppm, 79.7 ppm, 149.9 ppm, 159.3 ppm and 172.5 ppm in a solid state ¹³C NMR spectrum.
[36] Acrystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide hemioxalate, having a solid state ¹³C NMR spectrum substantially the same as the solid state ¹³C NMR spectrum shown in Fig. 9.
[37] A pharmaceutical composition comprising a salt according to [1] above or crystals according to any one of [2] to [36] above.
[38] An orexin type 2 receptor agonist comprising the salt according to [1] above or the crystal according to any one of [2] to [36] above.
[39] A therapeutic agent for narcolepsy comprising the salt according to [1] above or the crystal according to any one of [2] to [36] above.
[40] A treatment method for narcolepsy in a subject, which includes administration to the subject of a pharmacologically effective dose of the salt according to [1] above or the crystal according to any one of [2] to [36] above.
[41] A method for activating orexin type 2 receptor in a subject, which includes administration to the subject of a pharmacologically effective dose of the salt according to [1] above or the crystal according to any one of [2] to [36] above.
[42] A treatment method for narcolepsy, which includes administration to a subject of the salt according to [1] above or the crystal according to any one of [2] to [36] above.
[43] The use of the salt according to [1] above or the crystal according to any one of [2] to [36] above for production of a pharmaceutical composition for treatment of narcolepsy.
[44] The salt according to [1] above or the crystal according to any one of [2] to [36] above, to be used in treatment of narcolepsy.
[45] A therapeutic agent for cataplexy comprising the salt according to [1] above or the crystal according to any one of [2] to [36] above.
[46] A treatment method for cataplexy in a subject, which includes administration to the subject of a pharmacologically effective dose of the salt according to [1] above or the crystal according to any one of [2] to [36] above.
[47] The salt according to [1] above or the crystal according to any one of [2] to [36] above, to be used in treatment of cataplexy.
[48] The use of the salt according to [1] above or the crystal according to any one of [2] to [36] above for production of a pharmaceutical composition for treatment of cataplexy.
[49] A therapeutic agent for hypersomnia syndrome comprising the salt according to [1] above or the crystal according to any one of [2] to [36] above.
[50] A treatment method for hypersomnia syndrome in a subject, which includes administration of a pharmacologically effective dose of the salt according to [1] above or the crystal according to any one of [2] to [36] above.
[51] The salt according to [1] above or the crystal according to any one of [2] to [36] above, to be used in treatment of hypersomnia syndrome.
[52] The use of the salt according to [1] above or the crystal according to any one of [2] to [36] above for production of a pharmaceutical composition for treatment of hypersomnia syndrome.

### Advantageous Effects of Invention

According to the invention it is possible to provide crystals of compound (I), as well as salts of compound (I) and their crystals, having satisfactory properties which are expected to be useful as drug substances for pharmaceuticals.

### Brief Description of Drawings

Fig. 1 is an ORTEP diagram showing the results of X-ray crystallographic analysis of compound (I) obtained in Reference Example 1.
Fig. 2 is a powder X-ray diffraction pattern for Form α crystal of compound (I) obtained in Example 1. The abscissa represents diffraction angle (2θ) and the ordinate represents peak intensity.
Fig. 3 is a powder X-ray diffraction pattern for Form β crystal of compound (I) obtained in Example 2. The abscissa represents diffraction angle (2θ) and the ordinate represents peak intensity.
Fig. 4 is a powder X-ray diffraction pattern for crystal of compound (I) mono D-tartrate obtained in Example 3. The abscissa represents diffraction angle (2θ) and the ordinate represents peak intensity.
Fig. 5 is a powder X-ray diffraction pattern for crystal of compound (I) hemioxalate obtained in Example 4. The abscissa represents diffraction angle (2θ) and the ordinate represents peak intensity.
Fig. 6 is a solid state ¹³C NMR spectrum for Form α crystal of compound (I) obtained in Example 1. The abscissa represents chemical shift (δ) and the ordinate represents peak intensity.
Fig. 7 is a solid state ¹³C NMR spectrum for Form β crystal of compound (I) obtained in Example 2. The abscissa represents chemical shift (δ) and the ordinate represents peak intensity.
Fig. 8 is a solid state ¹³C NMR spectrum for crystal of compound (I) mono D-tartrate obtained in Example 3. The abscissa represents chemical shift (δ) and the ordinate represents peak intensity.
Fig. 9 is a solid state ¹³C NMR spectrum for crystal of compound (I) hemioxalate obtained in Example 4. The abscissa represents chemical shift (δ) and the ordinate represents peak intensity.
Fig. 10 is a thermal analysis TG-DTA chart for Form α crystal of compound (I) obtained in Example 1. The abscissa represents temperature, the left ordinate represents weight change of TG, and the right ordinate represents DTA heat flow rate.
Fig. 11 is a thermal analysis TG-DTA chart for Form β crystal of compound (I) obtained in Example 2. The abscissa represents temperature, the left ordinate represents weight change of TG, and the right ordinate represents DTA heat flow rate.
Fig. 12 is a thermal analysis TG-DTA chart for crystal of compound (I) mono D-tartrate obtained in Example 3. The abscissa represents temperature, the left ordinate represents weight change of TG, and the right ordinate represents DTA heat flow rate.
Fig. 13 is a thermal analysis TG-DTA chart for crystal of compound (I) hemioxalate obtained in Example 4. The abscissa represents temperature, the left ordinate represents weight change of TG, and the right ordinate represents DTA heat flow rate.
Fig. 14 is a Raman spectrum for Form α crystal of compound (I) obtained in Example 1.
Fig. 15 is a Raman spectrum for Form β crystal of compound (I) obtained in Example 2.
Fig. 16 is a graph showing hygroscopicity of Form α crystal of compound (I) obtained in Example 1.

### Description of Embodiments

The crystals of compound (I) and salts of compound (I) and their crystals, as well as their production processes, according to the present invention, will now be described in detail.

As used herein, "salt" means a chemical substance comprising compound (I) as the basic component and an acid in a specified number of equivalents with respect to compound (I).

The "salt" referred to herein may be an inorganic acid salt, an organic acid salt or an acidic amino acid salt, for example, and it is preferably a pharmaceutically acceptable salt.

Examples of salts of inorganic acids include salts of hydrochloric acid, hydrobromic acid, sulfuric acid, nitric acid and phosphoric acid, examples of salts of organic acids include salts of organic carboxylic acids such as acetic acid, succinic acid, fumaric acid, maleic acid, tartaric acid, malic acid, citric acid, lactic acid, stearic acid and benzoic acid, and salts of organic sulfonic acids such as methanesulfonic acid (mesylic acid), ethanesulfonic acid, benzenesulfonic acid and *p*-toluenesulfonic acid (tosylic acid), among which hydrochloric acid, hydrobromic acid and phosphoric acid are preferred.

Examples of salts of acidic amino acids include salts of aspartic acid and glutamic acid.

Compound (I) or its salt may also be an anhydride, hydrate or solvate. As used herein, "hydrate or solvate" refers to a solid formed by combination of compound (I) or its salt with water molecules or solvent molecules, the solid optionally being crystals, and examples of solvents for the solvate including ketone-based solvents such as acetone, 2-butanone and cyclohexanone; ester-based solvents such as methyl acetate and ethyl acetate; ether-based solvents such as 1,2-dimethoxyethane and t-butylmethyl ether; alcohol-based solvents such as methanol, ethanol, 1-propanol and isopropanol; and polar solvents such as N-methyl-2-pyrrolidone, N,N-dimethylformamide and dimethyl sulfoxide. The number of water molecules or solvent molecules with respect to compound (I) or its salt is not particularly restricted, and may be 1 or 2 molecules, for example.

The term "crystals" as used herein means crystals of compound (I) or its salt.

Preferred crystals of compound (I) or its salts for the purpose of the invention include:
Form α crystal of compound (I) having a diffraction peak at a diffraction angle (2θ ±0.2°) of 24.6° in a powder X-ray diffraction;
Form α crystal of compound (I) having diffraction peaks at diffraction angles (2θ ±0.2°) of 12.2°, 15.5° and 24.6° in a powder X-ray diffraction;
Form α crystal of compound (I) having diffraction peaks at diffraction angles (2θ ±0.2°) of 12.2°, 12.8°, 15.5°, 21.2° and 24.6° in a powder X-ray diffraction;
Form α crystal of compound (I) having diffraction peaks at diffraction angles (2θ ±0.2°) of 10.6°, 11.1°, 12.2°, 12.8°, 15.5°, 21.2°, 23.0°, 24.6°, 26.0° and 35.0° in a powder X-ray diffraction;
Form α crystal of compound (I) having a powder X-ray diffraction pattern substantially the same as the powder X-ray diffraction pattern shown in Fig. 2;
Form α crystal of compound (I) having a peak at a chemical shift (δ ±0.5 ppm) of 72.5 ppm in a solid state ¹³C NMR spectrum;
Form α crystal of compound (I) having peaks at chemical shifts (δ ±0.5 ppm) of 49.2 ppm, 67.6 ppm and 72.5 ppm in a solid state ¹³C NMR spectrum;
Form α crystal of compound (I) having peaks at chemical shifts (δ ±0.5 ppm) of 16.4 ppm, 49.2 ppm, 67.6 ppm, 72.5 ppm and 179.1 ppm in a solid state ¹³C NMR spectrum;
Form α crystal of compound (I) having peaks at chemical shifts (δ ±0.5 ppm) of 16.4 ppm, 27.7 ppm, 49.2 ppm, 55.9 ppm, 60.9 ppm, 67.6 ppm, 72.5 ppm, 144.4 ppm and 179.1 ppm in a solid state ¹³C NMR spectrum;
Form α crystal of compound (I) having a solid state ¹³C NMR spectrum substantially the same as the solid state ¹³C NMR spectrum shown in Fig. 6;
Form α crystal of compound (I) having a Raman shift peak (±2 cm⁻¹) at 1199.4 cm⁻¹ in Raman spectrophotometry;
Form α crystal of compound (I) having Raman shift peaks (±2 cm⁻¹) at 842.1 cm⁻¹, 895.0 cm⁻¹ and 1199.4 cm⁻¹ in Raman spectrophotometry;
Form α crystal of compound (I) having Raman shift peaks (±2 cm⁻¹) at 811.6 cm⁻¹, 842.1 cm⁻¹, 895.0 cm⁻¹, 1074.8 cm⁻¹ and 1199.4 cm⁻¹ in Raman spectrophotometry;
Form α crystal of compound (I) having Raman shift peaks (±2 cm⁻¹) at 508.1 cm⁻¹, 778.4 cm⁻¹, 811.6 cm⁻¹, 834.5 cm⁻¹, 842.1 cm⁻¹, 895.0 cm⁻¹, 934.9 cm⁻¹, 1074.8 cm⁻¹, 1094.2 cm⁻¹ and 1199.4 cm⁻¹ in Raman spectrophotometry;
Form α crystal of compound (I) having a Raman spectrum substantially the same as the Raman spectrum shown in Fig. 14;
Form β crystal of compound (I) having a diffraction peak at a diffraction angle (2θ ±0.2°) of 14.6° in a powder X-ray diffraction;
Form β crystal of compound (I) having diffraction peaks at diffraction angles (2θ ±0.2°) of 11.8°, 14.6° and 16.1° in a powder X-ray diffraction;
Form β crystal of compound (I) having diffraction peaks at diffraction angles (2θ ±0.2°) of 4.9°, 9.8°, 11.8°, 14.6° and 16.1° in a powder X-ray diffraction;
Form β crystal of compound (I) having diffraction peaks at diffraction angles (2θ ±0.2°) of 4.9°, 9.8°, 11.8°, 14.6°, 15.0°, 16.1°, 19.6°, 20.4°, 20.7° and 23.5° in a powder X-ray diffraction;
Form β crystal of compound (I) having a powder X-ray diffraction pattern substantially the same as the powder X-ray diffraction pattern shown in Fig. 3;
Form β crystal of compound (I) having a peak at a chemical shift (δ ±0.5 ppm) of 74.1 ppm in a solid state ¹³C NMR spectrum;
Form β crystal of compound (I) having peaks at chemical shifts (δ ±0.5 ppm) of 74.1 ppm, 159.2 ppm and 177.7 ppm in a solid state ¹³C NMR spectrum;
Form β crystal of compound (I) having peaks at chemical shifts (δ ±0.5 ppm) of 39.1 ppm, 70.6 ppm, 74.1 ppm, 159.2 ppm and 177.7 ppm in a solid state ¹³C NMR spectrum;
Form β crystal of compound (I) having peaks at chemical shifts (δ ±0.5 ppm) of 28.6 ppm, 39.1 ppm, 56.3 ppm, 60.4 ppm, 70.6 ppm, 74.1 ppm, 145.0 ppm, 150.5 ppm, 159.2 ppm and 177.7 ppm in a solid state ¹³C NMR spectrum;
Form β crystal of compound (I) having a solid state ¹³C NMR spectrum substantially the same as the solid state ¹³C NMR spectrum shown in Fig. 7;
Form β crystal of compound (I) having a Raman shift peak (±2 cm⁻¹) at 1089.4 cm⁻¹ in Raman spectrophotometry;
Form β crystal of compound (I) having Raman shift peaks (±2 cm⁻¹) at 889.9 cm⁻¹, 1089.4 cm⁻¹ and 1194.6 cm⁻¹ in Raman spectrophotometry;
Form β crystal of compound (I) having Raman shift peaks (±2 cm⁻¹) at 811.6 cm⁻¹, 837.0 cm⁻¹, 889.9 cm⁻¹, 1089.4 cm⁻¹ and 1194.6 cm⁻¹ in Raman spectrophotometry;
Form β crystal of compound (I) having Raman shift peaks (±2 cm⁻¹) at 716.6 cm⁻¹, 778.4 cm⁻¹, 811.6 cm⁻¹, 837.0 cm⁻¹, 889.9 cm⁻¹, 1035.9 cm⁻¹, 1072.4 cm⁻¹, 1089.4 cm⁻¹, 1194.6 cm⁻¹ and 1442.2 cm⁻¹ in Raman spectrophotometry;
Form β crystal of compound (I) having a Raman spectrum substantially the same as the Raman spectrum shown in Fig. 15;
crystal of compound (I) mono D-tartrate having a diffraction peak at a diffraction angle (2θ ±0.2°) of 4.1° in a powder X-ray diffraction;
crystal of compound (I) mono D-tartrate having diffraction peaks at diffraction angles (2θ ±0.2°) of 4.1°, 12.8° and 23.3° in a powder X-ray diffraction;
crystal of compound (I) mono D-tartrate having diffraction peaks at diffraction angles (2θ ±0.2°) of 4.1°, 12.8°, 16.1°, 20.0° and 23.3° in a powder X-ray diffraction;
crystal of compound (I) mono D-tartrate having diffraction peaks at diffraction angles (2θ ±0.2°) of 4.1°, 10.6°, 12.8°, 15.6°, 16.1°, 17.7°, 19.6°, 20.0°, 23.3° and 32.3° in a powder X-ray diffraction;
crystal of compound (I) mono D-tartrate having a powder X-ray diffraction pattern substantially the same as the powder X-ray diffraction pattern shown in Fig. 4;
crystal of compound (I) mono D-tartrate having a peak at a chemical shift (δ ±0.5 ppm) of 73.8 ppm in a solid state ¹³C NMR spectrum;
crystal of compound (I) mono D-tartrate having peaks at chemical shifts (δ ±0.5 ppm) of 71.6 ppm, 73.8 ppm and 179.3 ppm in a solid state ¹³C NMR spectrum;
crystal of compound (I) mono D-tartrate having peaks at chemical shifts (δ ±0.5 ppm) of 26.6 ppm, 71.6 ppm, 73.8 ppm, 174.1 ppm and 179.3 ppm in a solid state ¹³C NMR spectrum;
crystal of compound (I) mono D-tartrate having peaks at chemical shifts (δ ±0.5 ppm) of 13.2 ppm, 26.6 ppm, 42.3 ppm, 60.6 ppm, 62.0 ppm, 71.6 ppm, 73.8 ppm, 171.2 ppm, 174.1 ppm and 179.3 ppm in a solid state ¹³C NMR spectrum;
crystal of compound (I) mono D-tartrate having a solid state ¹³C NMR spectrum substantially the same as the solid state ¹³C NMR spectrum shown in Fig. 8;
crystal of compound (I) hemioxalate having a diffraction peak at a diffraction angle (2θ ±0.2°) of 10.0° in a powder X-ray diffraction;
crystal of compound (I) hemioxalate having diffraction peaks at diffraction angles (2θ ±0.2°) of 5.8°, 10.0° and 14.3° in a powder X-ray diffraction;
crystal of compound (I) hemioxalate having diffraction peaks at diffraction angles (2θ ±0.2°) of 5.0°, 5.8°, 10.0°, 13.2° and 14.3° in a powder X-ray diffraction;
crystal of compound (I) hemioxalate having diffraction peaks at diffraction angles (2θ ±0.2°) of 5.0°, 5.8°, 7.2°, 8.6°, 9.7°, 10.0°, 12.3°, 13.2°, 14.3° and 14.9° in a powder X-ray diffraction;
crystal of compound (I) hemioxalate having a powder X-ray diffraction pattern substantially the same as the powder X-ray diffraction pattern shown in Fig. 5;
crystal of compound (I) hemioxalate having a peak at a chemical shift (δ ±0.5 ppm) of 44.1 ppm in a solid state ¹³C NMR spectrum;
crystal of compound (I) hemioxalate having peaks at chemical shifts (δ ±0.5 ppm) of 27.3 ppm, 44.1 ppm and 62.4 ppm in a solid state ¹³C NMR spectrum;
crystal of compound (I) hemioxalate having peaks at chemical shifts (δ ±0.5 ppm) of 27.3 ppm, 44.1 ppm, 55.2 ppm, 62.4 ppm and 172.5 ppm in a solid state ¹³C NMR spectrum;
crystal of compound (I) hemioxalate having peaks at chemical shifts (δ ±0.5 ppm) of 27.3 ppm, 44.1 ppm, 55.2 ppm, 62.4 ppm, 67.5 ppm, 74.4 ppm, 79.7 ppm, 149.9 ppm, 159.3 ppm and 172.5 ppm in a solid state ¹³C NMR spectrum; and
crystal of compound (I) hemioxalate having a solid state ¹³C NMR spectrum substantially the same as the solid state ¹³C NMR spectrum shown in Fig. 9.

The diffraction peaks in powder X-ray diffraction, the chemical shifts in the solid state ¹³C NMR spectrum and the Raman shift peaks in Raman spectrophotometry mentioned above are unique for the Form α crystal of compound (I), the Form β crystal of compound (I), the crystal of compound (I) mono D-tartrate and the crystal of compound (I) hemioxalate, and they are the characteristic peaks for the respective crystals.

Since the diffraction angle (2θ) in powder X-ray diffraction is usually obtained at an error within a range of ±0.2°, the values for the aforementioned diffraction angles must be interpreted as including values within a range of about ±0.2°. Thus, the invention encompasses not only crystals having completely matching diffraction angles for peaks in powder X-ray diffraction, for specific compounds or their salts, but also identical crystals having peak diffraction angles that match with an error of about ±0.2°.

Throughout the present specification, the phrase "has a diffraction peak at a diffraction angle (2θ ±0.2°) of 24.6°", for example, has the same meaning as "has a diffraction peak at a diffraction angle (2θ) of 24.4° to 24.8°, and likewise for other diffraction angles.

Even with the same crystalline form, the measured peak intensity or half-width of a diffraction angle (2θ) in powder X-ray diffraction generally differs depending on differences in measuring conditions and the sizes and shapes of particles in the powder crystals used as the measuring sample, and a consistent peak intensity or half-width is not necessary always exhibited. In comparison of powder X-ray diffraction patterns, therefore, differences in peak intensity or half-width at the same diffraction angle (2θ) do not mean that they derive from different crystalline forms. Therefore a powder X-ray diffraction pattern with such differences from the characteristic diffraction peak for a specific crystal of the invention signifies the same crystalline form as the crystal of the invention. The phrase "having the powder X-ray diffraction pattern of Fig. 2" as used herein includes not only cases where a powder X-ray diffraction pattern having a characteristic diffraction peak matches the powder X-ray diffraction pattern shown in Fig. 2 within a margin of error of ±0.2°, but also powder X-ray diffraction patterns wherein the characteristic diffraction angle matches within a margin of error of ±0.2° but with differences in the peak intensity or half-width, and even in such cases, all crystals exhibiting the powder X-ray diffraction pattern shown in Fig. 2 are the same crystals as the crystals of the invention.

References herein similar to "chemical shifts (δ ±0.5 ppm) of 27.7 ppm, 49.2 ppm, 55.9 ppm, 67.6 ppm and 72.5 ppm" mean peaks having chemical shifts (δ ±0.5 ppm) substantially identical to 27.7 ppm, 49.2 ppm, 55.9 ppm, 67.6 ppm and 72.5 ppm respectively, when the solid state ¹³C NMR spectrum is measured under conditions which are substantially the same as ordinary measuring conditions or the conditions described herein.

Since a chemical shift δ in the solid state ¹³C NMR spectrum may exhibit error in the range of ±0.5 ppm, determining whether or not peaks are "substantially identical" generally requires the understanding that the value of the chemical shift also includes values in the range of about ±0.5 ppm. Thus, crystals that do not have completely matching chemical shifts in the solid state ¹³C NMR spectrum, but have chemical shifts matching within a range of error of about ±0.5 ppm, are also included within the scope of the invention. Consequently, a reference herein to "having a chemical shift (δ ±0.5 ppm) of 16.4 ppm", for example, means a peak having a chemical shift (δ) in the range of 15.9 ppm to 16.9 ppm, and the same applies to other chemical shifts in the solid state ¹³C NMR spectrum.

The Raman shift peak (cm⁻¹) in Raman spectrophotometry generally has an error range of ±2 cm⁻¹, and therefore the peak values should be understood as including values in the range of about ±2 cm⁻¹. For the specified compounds and their salts, therefore, not only crystals with completely matching Raman shift peaks in Raman spectrophotometry but also crystals with Raman shift peaks matching within a range of error of about ±2 cm⁻¹, are also within the scope of the invention.

The phrase "having a Raman shift peak (±2 cm⁻¹) at 1199.4 cm⁻¹ in Raman spectrophotometry", for example, as used herein, means "having a Raman shift peak at 1197.4 cm⁻¹ to 1201.4 cm⁻¹ in Raman spectrophotometry", and the same applies to other Raman shifts as well.

Even with the same crystalline form, the measured peak intensity or half-width of a Raman shift in Raman spectrophotometry generally differs depending on differences in measuring conditions and the sizes and shapes of particles in the powder crystals used as the measuring sample, and a consistent peak intensity or half-width is not necessary always exhibited. For comparison of measurements in Raman spectrophotometry, therefore, differences in peak intensity or half-width at the same Raman shift peak (cm⁻¹) do not mean that they derive from different crystalline forms. Therefore a Raman spectrum with such differences from the characteristic Raman shift peak for a specific crystal of the invention signifies the same crystalline form as the crystal of the invention. A reference to "having the spectrum of Fig. 14 in Raman spectrophotometry" as used herein includes not only cases where a Raman spectrum having a characteristic Raman shift peak (cm⁻¹) matches the Raman spectrum shown in Fig. 14 within a margin of error of ±2 cm⁻¹, but also Raman spectra wherein the characteristic Raman shift peak matches within a margin of error of ±2 cm⁻¹ even with differences in peak intensity or half-width, and even in such cases, all crystals exhibiting the Raman spectrum shown in Fig. 14 are the same crystals as the crystals of the invention.

Processes for producing salts and crystals of compound (I), as one embodiment of the invention, will now be described.

### Processes for producing compound (I)

Compound (I) may be one produced by a process known to those skilled in the art. For example, compound (I) can be synthesized by the process described in the Reference Example below.

### Processes for producing salts of compound (I)

Salts of compound (I) of the invention may be produced by common processes for producing salts. As a specific example, compound (I) may be suspended or dissolved in a solvent, with heating if necessary, and an acid may then be added to the obtained suspension or solution prior to stirring or standing for several minutes to several days at room temperature or with cooling. Such a production process can yield a salt of compound (I) as crystals or in amorphous form. As necessary, an amorphous form may also be further processed in such methods by freeze-drying, for example. The solvent to be used may be, for example, an alcohol-based solvent such as ethanol, 1-propanol or isopropanol; acetonitrile; a ketone-based solvent such as acetone or 2-butanone; an ester-based solvent such as ethyl acetate; a saturated hydrocarbon-based solvent such as hexane or heptane; an ether-based solvent such as t-butylmethyl ether, or water. These solvents may be used alone, or two or more different ones may be used in admixture.

Process for producing crystals of compound (I) or its salt Crystals of compound (I) or its salt can be produced by the process for producing compound (I) described above, or a process for producing its salt, or compound (I) or its salt may be heated and dissolved in a solvent and crystallized by cooling while stirring.

Compound (I) or its salt to be used for crystallization may be in any prepared form such as a solvate, hydrate or anhydride, and it may be either amorphous or crystalline (including multiple polymorphic crystals), or mixtures of these.

The solvent to be used for crystallization may be an alcohol-based solvent such as methanol, ethanol, isopropanol or 1-propanol; acetonitrile; an amide-based solvent such as N,N-dimethylformamide; an ester-based solvent such as ethyl acetate; a saturated hydrocarbon-based solvent such as hexane or heptane; a ketone-based solvent such as acetone or 2-butanone; an ether-based solvent such as t-butylmethyl ether; or water. These solvents may be used alone, or two or more different ones may be used in admixture.

The amount of solvent used may be appropriately selected, with the lower limit as an amount which allows compound (I) or its salt to dissolve by heating or an amount that allows stirring of the suspension, and the upper limit as an amount which does not notably reduce the crystal yield.

For crystallization, seed crystals may be added (for desired crystals of compound (I) or its salt), but they do not need to be added. The temperature for adding seed crystals is not particularly restricted but is preferably 0 to 80°C.

The temperature for dissolution of compound (I) or its salt by heating may be selected as an appropriate temperature that dissolves compound (I) or its salt in the solvent used, but it is preferably in a range from 50°C to the temperature at which the recrystallization solvent begins to undergo reflux, and it is more preferably 55 to 80°C.

Since quenching can result in inclusion of crystals of different types (multiform crystals), the cooling during crystallization is preferably carried out at an appropriate cooling rate in consideration of effects on the quality and particle sizes of the crystals, and it is preferably cooling at a rate of 5 to 40°C/hour, for example. The cooling rate is more preferably 5 to 25°C/hour, for example.

The final crystallization temperature may be appropriately selected based on the salt yield and quality, but it is preferably -25 to +30°C.

The formed crystals are separated by an ordinary filtration procedure, and if necessary the filtered crystals are rinsed with a solvent and dried to obtain the desired crystals. The solvent used for rinsing of the crystals may be the same as the crystallization solvent. Preferred examples include ethanol, acetone, 2-butanone, ethyl acetate, diethyl ether, t-butylmethyl ether and hexane. These solvents may be used alone, or two or more different ones may be used in admixture.

The crystals separated by the filtration procedure may be appropriately dried by standing under air or a nitrogen stream, or by heating.

The drying time may be appropriately selected to be a time at which the residual solvent falls below a prescribed volume, and this will depend on the production volume, the drying apparatus and the drying temperature. The drying may be carried out with ventilation or under reduced pressure. The degree of pressure reduction may be appropriately selected depending on the production volume, the drying apparatus and the drying temperature. The obtained crystals may be dried and then left to stand in air if necessary.

Crystals of compound (I) and salts of compound (I) obtained by the production process described above have orexin type 2 receptor agonist activity, as demonstrated by the activity data in the Pharmacological Test Examples described below, and they have potential as therapeutic agents for narcolepsy, for example.

### [Pharmaceutical composition]

Another embodiment of the invention is a pharmaceutical composition comprising crystals of compound (I) and a pharmaceutically acceptable additive. The pharmaceutical composition can be produced by mixing a pharmaceutically acceptable additive with crystals of compound (I). The pharmaceutical composition of the invention can be produced by a known method, such as the method described in "General Rules for Preparations" of the Japanese Pharmacopoeia, 17th Edition.

The pharmaceutical composition of the embodiment may be appropriately administered to a patient in a manner suitable for the dosage form.

The dosage of compound (I) according to the invention may vary depending on the severity of symptoms, age, gender and body weight of the patient, the dosage form or the type of salt, and the specific type of disease, but it will usually be about 30 µg to 10 g, preferably 100 µg to 5 g and even more preferably 100 µg to 1 g for oral administration, and about 30 µg to 1 g, preferably 100 µg to 500 mg and even more preferably 100 µg to 300 mg for administration by injection, as the dosage per day for an adult, either at once or in divided doses.

### Examples

Crystals of compound (I) of the invention may be produced by the processes described in the following Examples, and the effects exhibited by the compounds may be confirmed by the methods described in the following Test Examples. However, these Examples are merely illustrative and are not intended to restrict the invention in any way, while various modifications may also be implemented such as are within the scope of the invention.

For powder X-ray crystal diffraction of the crystals produced in the following Examples, the obtained crystals were placed on the sample stage of the powder X-ray diffraction apparatus and analyzed under the following conditions.

### (Transmission conditions)

X-ray source: Cu-Kα
Voltage: 45 kV
Current: 200 mA
Optical system: Focusing optical system
Soller slit: 2.5°
Detector: D/teX Ultra 250 (1D semiconductor detector)
Scan speed: 10°/min
Step width: 0.01°
Scan range: 3°-40°
Sample holder: Aluminum holder and Mylar film

Thermal analysis was carried out by precisely weighing out the sample onto an aluminum sample pan and performing measurement under the following conditions.

### (Measuring conditions)

Atmosphere: Nitrogen gas stream at 100 mL/min
Control: Empty aluminum sample pan
Temperature-elevating rate: 10°C/min
Sampling interval: 1 sec
Measuring temperature range: Room temperature to 250°C or room temperature to 200°C

For solid state ¹³C NMR spectrum of the crystals, 200 to 300 mg of solid sample was filled into a sample tube and measurement was performed under the following conditions.

### (Measuring conditions)

Apparatus: Avance 400 MHz (Bruker Co.), 7 mm-CPMAS probe (Bruker Co.)
Measured nucleus: ¹³C (100.6248425 MHz)
Measuring temperature: Room temperature
Pulse mode: CPTOSS
Rotational speed: 5000 Hz
Pulse repetition time: 5 sec
Contact time: 1 msec
Number of scans: 5120 or 10,240
Reference substance: Glycine (external reference: 176.03 ppm)

For Raman spectrum of the crystals, 50 to 100 mg of solid sample was filled into a measuring plastic bag and measurement was performed under the following conditions.

### (Measuring conditions)

Apparatus: TRS100 Raman (Agilent)
Laser wavelength: 830 nm
Laser output: 0.65 W
Exposure time: 1.000 sec
Number of scans: 10 (Form α) or 20 (Form β)
Laser spot size: 4 mm
Collection size: Medium

Compounds mentioned herein with reference to published documents were produced in the manner described in those documents.

The abbreviations used throughout are those commonly known among those skilled in the art. In particular, the following are used.
*n*-: normal
*tert-*:tertiary
¹H-NMR: Proton Nuclear Magnetic Resonance spectrometry
MS: Mass Spectrometry
HPLC: High-Performance Liquid Chromatography

The term "room temperature" used throughout the Examples and Reference Examples generally refers to a range of about 10°C to 35°C. The percentage values are weight percentages, unless otherwise specified.

The chemical shifts in the proton nuclear magnetic resonance spectra are recorded in δ units (ppm) with respect to tetramethylsilane, and the coupling constants are recorded in Hertz (Hz). The patterns are represented as s: singlet, d: doublet, t: triplet, q: quartet, m: multiplet, br: broad and brs: broad singlet.

Mass spectrometry was carried out using an Acquity UPLC^{R} or Acquity UPC² by Waters Co.

For chromatography, Silica Gel60 by Merck (70-230 mesh or 230-400 mesh ASTM) or PSQ60B by Fuji Silysia Chemical, Ltd. was used as the silica gel, or a prepacked column (column: Hi-Flash^{™} Column (Silicagel) by Yamazen, size: S (16 × 60 mm), M (20 × 75 mm), L (26 × 100 mm), 2 L (26 × 150 mm) or 3 L (46 × 130 mm), or a Biotage^{™} SNAP Ultra Silica Cartridge by Biotage Co., size: 10 g, 25 g or 50 g) was used. Fractionation by supercritical fluid chromatography (SFC) was carried out using a Prep100q by Waters Co.

As NH silica gel, either CHROMATOREX NH-DM2035 by Fuji Silysia Chemical, Ltd. was used, or a prepacked column (column: Hi-Flash^{™} Column (Amino) by Yamazen, size: S (16 × 60 mm), M (20 × 75 mm), L (26 × 100 mm), 2 L (26 × 150 mm) or 3 L (46 × 130 mm), or Presep^{™} (Luer Lock) NH₂(HC) by Wako Pure Chemical Industries, Ltd., size: type M (14 g/25 mL), type L (34 g/70 mL), type 2 L (50 g/100 mL) or type 3 L (110 g/200 mL)).

The nomenclature used for the following compounds is the same indicated in "E-Notebook" version 12 or 13 (Perkin-Elmer), except for commonly used reagents.

### Reference Example 1

### Synthesis of 2-bromo-2-cyclopropyl acetamide

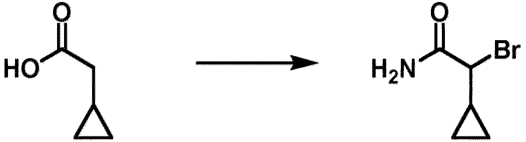

Oxalyl chloride (CAS No. 79-37-8) (3.11 mL, 36.3 mmol) and N,N-dimethylformamide (60.0 µL, 0.775 mmol) were added to a solution of cyclopropylacetic acid (CAS No. 5239-82-7) (3.30 g, 33.0 mmol) in 1,2-dichloroethane (60 mL), and the mixture was stirred for 40 minutes at room temperature. Hydrobromic acid (56.0 mg, 0.330 mmol) and N-bromosuccinimide (CAS No. 128-08-5) (7.04 g, 39.6 mmol) were added to the reaction mixture, which was then heated to reflux for 18 hours. The reaction mixture was added to ammonia (28% aqueous solution, 60 mL, 2.77 mol) at 0°C, and then ethyl acetate and sodium hydroxide (2 N aqueous solution) were added for separation. The organic layer was dried over anhydrous magnesium sulfate, filtered and concentrated under reduced pressure. The resulting solid was triturated with ethyl acetate/n-heptane and the precipitate was filtered out. The resulting solid was dried under reduced pressure to obtain the title compound (1.20 g).
MS (ESI)m/z: 178[M+H]⁺

### Reference Example 2

### Synthesis of 3-methoxyisonicotinonitrile

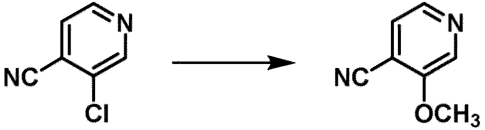

Sodium methoxide (CAS No. 124-41-4) (3.90 g, 72.2 mmol) was added to a solution of 3-chloro-4-cyanopyridine (CAS No. 68325-15-5) (5.00 g, 36.1 mmol) in tetrahydrofuran (36.0 mL) at room temperature, and the mixture was heated to reflux for 1 hour. Water and ethyl acetate were added to the reaction mixture, and the organic layer was separated off. The organic layer was dried over anhydrous magnesium sulfate, filtered and concentrated under reduced pressure. The resulting solid was triturated with ethyl acetate/*n*-heptane and the precipitate was filtered out. The resulting solid was dried under reduced pressure to obtain the title compound (1.91 g).

¹H-NMR (400 MHz, CDCl₃) δ(ppm): 4.06 (s, 3H), 7.44 (d, J = 5.0 Hz, 1H), 8.37 (d, J = 4.5 Hz, 1H), 8.49 (s, 1H).
MS (ESI)m/z: 135[M+H]⁺

### Reference Example 3

### Synthesis of (R)-2-bromo-3-methylbutaneamide

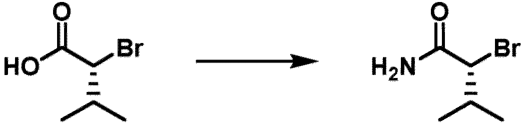

Oxalyl chloride (9.28 mL, 108 mmol) and N,N-dimethylformamide (30.0 µL, 0.387 mmol) were added to a solution of (R)-2-bromo-3-methylbutyric acid (CAS No. 76792-22-8) (9.80 g, 54.1 mmol) in methylene chloride (100 mL) at 0°C, and the mixture was stirred for 6 hours at room temperature. The reaction mixture was added to ammonia (28% aqueous solution, 50.0 mL, 2.31 mol) at 0°C, and then extracted with methylene chloride. The organic layer was dried over anhydrous magnesium sulfate, filtered and concentrated under reduced pressure. The resulting solid was triturated with ethyl acetate/n-heptane and the precipitate was filtered out. The resulting solid was dried under reduced pressure to obtain the title compound (8.20 g).
¹H-NMR (400 MHz, CDCl₃) δ(ppm): 1.01 (d, J = 6.3 Hz, 3H), 1.07 (d, J = 6.8 Hz, 3H), 2.34-2.39 (m, 1H), 4.28 (d, J = 4.5 Hz, 1H), 5.58 (brs, 1H), 6.41(brs, 1H). MS (ESI)m/z: 180[M+H]⁺

### Reference Example 4

### Synthesis of (1R,3s,5S)-3-((3S,4R)-1-(5-fluoropyrimidin-2-yl)-3-methoxypiperidin-4-yl)-8-azabicyclo[3.2.1]octane

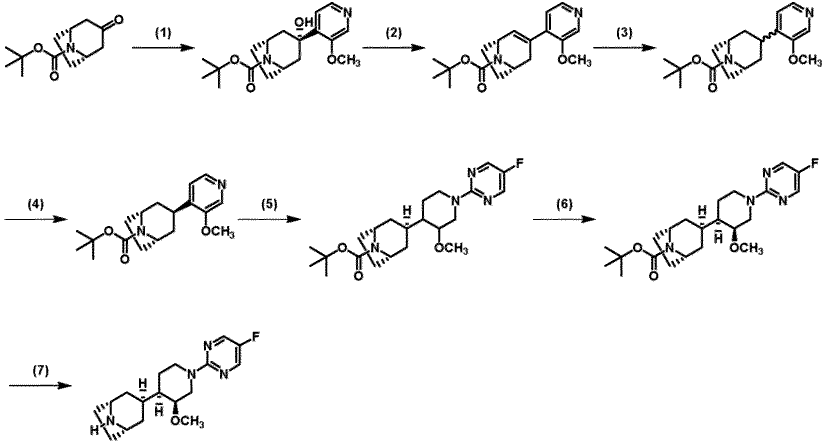

### (1) Synthesis of tert-butyl (1R,3r,5S)-3-hydroxy-3-(3-methoxypyridin-4-yl)-8-azabicyclo[3.2.1]octane-8-carboxylate

After adding 3-methoxyisonicotinonitrile (1.19 g, 8.88 mmol) and bis(pinacolato)diboron (CAS No. 73183-34-3) (2.25 g, 8.88 mmol) to a solution of N-(*tert*-butoxycarbonyl)-nortropinone (CAS No. 185099-67-6) (1.00 g, 4.44 mmol) in methyl *tert*-butyl ether (18.0 mL), the mixture was heated to reflux for 16 hours. Sodium carbonate (2 mol/L aqueous solution, 20.0 mL) was added to the reaction mixture at 0°C, and the mixture was stirred for 20 minutes at 0°C. Ethyl acetate was added to the reaction mixture and the organic layer was separated off. The organic layer was dried over anhydrous magnesium sulfate, filtered and concentrated under reduced pressure. The residue was purified by column chromatography (silica gel, 0-20% methanol/ethyl acetate) to obtain the title compound (1.12 g).

¹H-NMR (400 MHz, CDCl₃) δ(ppm): 1.48 (s, 9H), 1.77 (m, 2H), 1.94-1.99 (m, 2H), 2.21-2.32 (m, 2H), 2.37-2.48 (m, 1H), 2.63-2.74 (m, 1H), 2.78 (s, 1H), 3.96 (s, 3H), 4.22-4.29 (m, 1H), 4.31-4.42 (m, 1H), 7.28 (d, J = 5.4 Hz, 1H), 8.22-8.25 (m, 2H).
MS (ESI)m/z: 335[M+H]⁺

### (2) Synthesis of tert-butyl (1R,5S)-3-(3-methoxypyridin-4-yl)-8-azabicyclo[3,2.1]oct-2-ene-8-carboxylate

Concentrated sulfuric acid (1.60 mL, 30.0 mmol) was added to *tert*-butyl (1R,3r,5S)-3-hydroxy-3-(3-methoxypyridin-4-yl)-8-azabicyclo[3.2.1]octane-8-carboxylate (610 mg, 1.82 mmol), and the mixture was stirred for 40 minutes at room temperature. The reaction mixture was added to a solution of potassium hydroxide (5.00 g, 89.1 mmol) in water (10.0 mL) at 0°C. Tetrahydrofuran (10.0 mL) and di-*tert*-butyl dicarbonate (CAS No. 24424-99-5) (478 mg, 2.19 mmol) were added to the reaction mixture, which was then stirred for 10 minutes at room temperature. Ethyl acetate was added to the reaction mixture and the organic layer was separated off. The organic layer was dried over anhydrous magnesium sulfate, filtered and concentrated under reduced pressure. The residue was purified by column chromatography (silica gel, 5%-100% ethyl acetate/n-heptane) to obtain the title compound (420 mg).

¹H-NMR (400 MHz, CDCl₃) δ(ppm): 1.46 (s, 9H), 1.72-1.81 (m, 1H), 1.97-2.03 (m, 2H), 2.05-2.36 (m, 2H), 2.94-3.26 (m, 1H), 3.87 (s, 3H), 4.21-4.61 (m, 2H), 6.30(brs, 1H), 6.99 (d, J = 4.5 Hz, 1H), 8.17 (d, J = 5.0 Hz, 1H), 8.21 (s, 1H).
MS (ESI)m/z: 317[M+H]⁺

### (3) Synthesis of tert-butyl (1R,5S)-3-(3-methoxypyridin-4-yl)-8-azabicyclo[3.2.1]octane-8-carboxylate

After adding 10% palladium-carbon (AD, 52.7% aqueous, 284 mg, 0.126 mmol, product of Kawaken Fine Chemicals Co., Ltd.) to a solution of *tert*-butyl (1R,5S)-3-(3-methoxypyridin-4-yl)-8-azabicyclo[3.2.1]oct-2-ene-8-carboxylate (400 mg, 1.26 mmol) in methanol (3.00 mL), the mixture was stirred for 1 hour at room temperature under a hydrogen atmosphere. The reaction mixture was filtered with Celite^{R} and the residue was rinsed with ethyl acetate. The obtained filtrate was concentrated under reduced pressure to obtain a mixture of the endo form and exo form (endo:exo = 1:2, 398 mg).

¹H-NMR (400 MHz, CDCl₃) δ(ppm): 1.49 (s, 6H), 1.50 (s, 3H), 1.61-2.13 (m, 22/3H), 2.40-2.45 (m, 2/3H), 2.96-3.01 (m, 1/3H), 3.47-3.59 (m, 2/3H), 3.88 (s, 1H), 3.90 (s, 2H), 4.16-4.28 (m, 1H), 4.34 (brs, 1H), 7.04 (d, J = 4.5 Hz, 2/3H), 7.06 (d, J = 5.0 Hz, 1/3H), 8.13-8.23 (m, 2H).
MS (ESI)m/z: 319[M+H]⁺

### (4) Synthesis of tert-butyl (1R,3s,5S)-3-(3-methoxypyridin-4-yl)-8-azabicyclo[3.2.1]octane-8-carboxylate

Potassium *tert*-butoxide (281 mg, 2.50 mmol) was added to a solution of *tert*-butyl (1R,SS)-3-(3-methoxypyridin-4-yl)-8-azabicyclo[3.2.1]octane-8-carboxylate (398 mg, 1.25 mmol) in *tert*-butanol (4.00 mL), and the mixture was heated to reflux for 17 hours. Ethyl acetate and brine were added to the reaction mixture and the organic layer was separated off. The organic layer was then dried over anhydrous magnesium sulfate, filtered and concentrated under reduced pressure to obtain the title compound (385 mg).

¹H-NMR (400 MHz, CDCl₃) δ(ppm): 1.49 (s, 9H), 1.58-2.06 (m, 8H), 3.49-3.56 (m, 1H), 3.90 (s, 3H), 4.24 (brs, 1H), 4.34 (brs, 1H), 7.04 (d, J = 5.0 Hz, 1H), 8.17-8.19 (m, 2H).
MS (ESI)m/z: 319[M+H]⁺

### (5) Synthesis of tert-butyl (1R3s,5S)-3-(1-(5-fluoropyrimidin-2-yl)-3-methoxypiperidin-4-yl)-8-azabicyclo[3.2.1]octane-8-carboxylate

After adding 10% palladium-carbon (AD, 52.7% aqueous, 7.91 g, 3.52 mmol, product of Kawaken Fine Chemicals Co., Ltd.) to a solution of *tert*-butyl (1R,3s,5S)-3-(3-methoxypyridin-4-yl)-8-azabicyclo[3.2.1]octane-8-carboxylate (11.2 g, 35.2 mmol) in acetic acid (100 mL), the mixture was stirred for 18 hours at 70°C under a hydrogen atmosphere. The reaction mixture was filtered with Celite^{R} and the residue was rinsed with ethyl acetate. The filtrate was concentrated under reduced pressure. Ethyl acetate and sodium hydroxide (2 N) were added to the residue, and the organic layer was separated off. The organic layer was dried with ISOLUTE^{R} HM-N, filtered and concentrated under reduced pressure. After adding N,N-dimethylformamide (50.0 mL), 2-chloro-5-fluoropyrimidine (CAS No. 62802-42-0) (5.21 mL, 42.2 mmol) and potassium carbonate (7.29 g, 52.8 mmol) to the residue, the mixture was stirred for 40 minutes at 80°C. Ethyl acetate and brine were added to the reaction mixture and the organic layer was separated off. The organic layer was dried over anhydrous magnesium sulfate, filtered and concentrated under reduced pressure. The residue was purified by column chromatography (silica gel, 10-60% ethyl acetate/n-heptane) to obtain the title compound (9.84 g).

¹H-NMR (400 MHz, CDCl₃) δ(ppm): 1.19-1.30 (m, 3H), 1.46 (s, 9H), 1.46-1.65 (m, 6H), 1.81-1.97 (m, 3H), 2.68 (d, J = 14.5 Hz, 1H), 2.71-2.81 (m, 1H), 3.30 (s, 3H), 3.35(brs, 1H), 4.08-4.31 (m, 2H), 4.64-4.77 (m, 1H), 5.04-5.18 (m, 1H), 8.13 (s, 2H).
MS (ESI)m/z: 421[M+H]⁺

### (6) Synthesis of tert-butyl (1R,3s,5S)-3-((3S,4R)-1-(5-fluoropyrimidin-2-yl)-3-methoxypiperidin-4-yl)-8-azabicyclo[3.2.1]octane-8-carboxylate

The compound *tert*-butyl (1R,3s,5S)-3-(1-(5-fluoropyrimidin-2-yl)-3-methoxypiperidin-4-yl)-8-azabicyclo[3.2.1]octane-8-carboxylate (7.00 g, 16.6 mmol) was fractionated 100 mg at a time by supercritical fluid chromatography using CHIRALPAK^{R} IC/SFC (3 cm × 25 cm) by Daicel (mobile phase: CO₂:methanol (90:10), 120 bar, 40°C, flow rate: 100 mL/min), obtaining the subsequently eluting title compound (3.02 g) at a retention time of 8.45 minutes. ¹H-NMR (400 MHz, CDCl₃) δ(ppm): 1.17-1.29 (m, 3H), 1.46 (s, 9H), 1.46-1.66 (m, 6H), 1.85-1.97 (m, 3H), 2.68 (d, J = 15.0 Hz, 1H), 2.75 (td, J = 12.9,3.2 Hz, 1H), 3.30 (s, 3H), 3.35(brs, 1H), 4.11-4.31 (m, 2H), 4.66-4.76 (m, 1H), 5.10 (dt, J = 14.4, 2.6 Hz, 1H), 8.13 (s, 2H).
MS (ESI)m/z: 443[M+Na]⁺

(Analysis conditions) Supercritical fluid chromatography using CHIRALPAK^{R} IC-3 (3.0 mm × 50 mm) by Daicel (mobile phase: CO₂:methanol (85:15), 40°C, flow rate: 1.2 mL/min, detection: UV (254 nm)).

(Analysis results) The retention time of the title compound was 1.34 minutes and the optical purity was >99% ee.

### (7) Synthesis of (1R,3s,5S)-3-((3S,4R)-1-(5-fluoropyrimidin-2-yl)-3-methoxypiperidin-4-yl)-8-azabicyclo[3.2.1]octane

Trifluoroacetic acid (5.00 mL, 64.9 mmol) was added to *tert*-butyl (1R,3s,5S)-3-((3 S,4R)-1-(5-fluoropyrimidin-2-yl)-3-methoxypiperidin-4-yl)-8-azabicyclo[3.2.1]octane-8-carboxylate (1.00 g, 2.38 mmol), and the mixture was stirred for 20 minutes at room temperature. The reaction mixture was concentrated under reduced pressure. Aqueous saturated 2 N sodium hydroxide was added to the residue and extraction was performed with ethyl acetate (3 times). The combined organic layers were dried over anhydrous magnesium sulfate, filtered and concentrated under reduced pressure to obtain the title compound (620 mg).
MS (ESI)m/z: 321[M+H]⁺

### Reference Example 5

### Synthesis of (S)-2-bromo-2-cyclopropyl acetamide

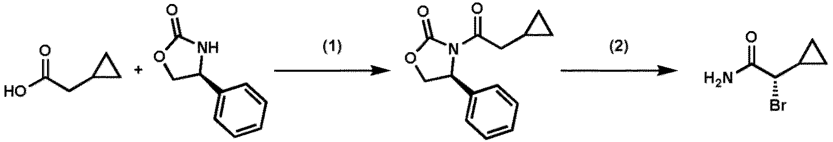

### (1) Synthesis of (S)-3-(2-cyclopropylacetyl)-4-phenyloxazolidin-2-one

Pivaloyl chloride (302 mL, 2470 mmol) was added to tetrahydrofuran (7000 mL) at room temperature. Cyclopropylacetic acid (238 mL, 2450 mmol) was added at room temperature and the mixture was cooled to 0°C. After dropwise addition of triethylamine (350 mL) over a period of 10 minutes, triethylamine (400 mL, total amount: 5340 mmol) was added and the mixture was stirred for 76 minutes at 0°C. After then adding (S)-4-phenyloxazolidin-2-one (350 g, 2140 mmol) to the reaction mixture all at once, lithium chloride (109 g, 2570 mmol) was further added all at once. The reaction mixture was stirred for 18 hours at room temperature, and then ethyl acetate (7000 mL) and water (3500 mL) were added and the mixture was stirred for 40 minutes at room temperature. The organic layer was separated and rinsed with aqueous 5% sodium hydrogencarbonate (3500 mL) and water (1750 mL), in that order. The obtained organic layer was concentrated to 1750 mL. An azeotropic procedure of adding ethyl acetate (2100 mL) and concentrating to 1750 mL was repeated 3 times, after which ethyl acetate (1050 mL) was added and the mixture was concentrated to 1750 mL. The obtained concentrate was stirred, and *n-*heptane (1000 mL) was added dropwise. The suspension that was produced was stirred for 10 minutes, and *n-*heptane (2500 mL) was further added dropwise. The liquid mixture was stirred overnight at room temperature, and then further stirred for 3.5 hours at 0°C. The produced solid was filtered using a glass filter and rinsed with ethyl acetate/*n*-heptane (550 mL of a 1:3 mixture). The resulting solid was dried under reduced pressure to obtain the title compound (407 g).

¹H-NMR (400 MHz, CDCl₃) δ(ppm): 0.09-0.24 (m, 2H)0.46-0.58 (m, 2H)1.00-1.13 (m, 1H), 2.74-2.83 (m, 1H), 2.88-2.98 (m, 1H), 4.25-4.32 (m, 1H), 4.70 (t, J = 8.83 Hz, 1H), 5.45 (dd, J = 8.83, 3.85 Hz, 1H), 7.28-7.43 (m, 5H).

### (2) Synthesis of (S)-2-bromo-2-cyclopropyl acetamide

A 1 M dichloromethane solution of dibutylboron triflate (500 mL, 500 mmol) was added dropwise to a solution of (S)-3-(2-cyclopropylacetyl)-4-phenyloxazolidin-2-one (100 g, 408 mmol) in dichloromethane (1000 mL) over a period of 40 minutes while cooling on ice, and the mixture was stirred for 10 minutes while cooling on ice. After adding N,N-diisopropylethylamine (92 mL, 530 mmol) dropwise over a period of 25 minutes while cooling on ice, the mixture was stirred for 1 hour while cooling on ice. The reaction mixture was then cooled to an internal temperature of - 72°C in a dry ice-ethanol bath. Next, N-bromosuccinimide (80 g, 448 mmol) was added all at once, and the mixture was stirred for 1 hour and 20 minutes in a dry ice-ethanol bath. After adding 28-30% ammonia water (800 mL, 6390 mmol) and tetrahydrofuran (1000 mL), the mixture was stirred for 2 hours in a water bath. The organic layer and aqueous layer were separated, and the aqueous layer was extracted 3 times with ethyl acetate (500 mL). The obtained organic layer was concentrated under reduced pressure, and the obtained residue was purified by silica gel column chromatography (4 kg silica gel, 30-40% ethyl acetate/*n*-heptane) to obtain 132 g of a crude product. After adding *tert*-butyl methyl ether (1440 mL) to the obtained crude product and stirring for 1 hour at 50°C to dissolution, the mixture was further stirred for 1 day at room temperature. The resulting solid was filtered and rinsed with *tert*-butyl methyl ether (200 mL). The filtrate and rinse solution were combined, ethyl acetate (700 mL) and active carbon (SEISEI SHIRASAGI, 26 g) were added and the mixture was stirred for 30 minutes at room temperature. The active carbon was removed by Celite^{R} filtration, and the active carbon was rinsed with ethyl acetate (700 mL). After combining the filtrate and rinse solution, the mixture was concentrated under reduced pressure to obtain the title compound (97.1 g, 65.9% content).

¹H-NMR (400 MHz, CDCl₃) δ(ppm): 0.40-0.52 (m, 1H), 0.63-0.73 (m, 1H), 0.77-0.86 (m, 1H), 0.86-0.97 (m, 1H), 1.41-1.50 (m, 1H), 3.59-3.83 (m, 1H), 5.34-5.71 (m, 1H), 5.94-6.30 (m, 1H).
MS (ESI)m/z: 180[M+H]⁺

(Analysis conditions) Chromatography using CHIRALPAK^{R} IA (0.46 cm × 25 cm × 2) by Daicel (mobile phase: ethanol:n-hexane (10:90), 40°C, flow rate: 0.8 mL/min, detection: UV (210 nm)).

(Analysis results) The retention time of the title compound was 24.5 minutes.

### Reference Example 6

### Synthesis of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide

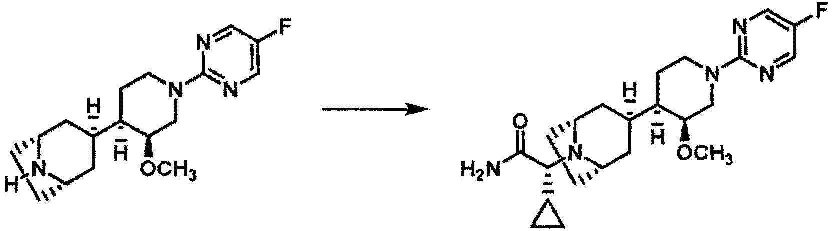

After adding 2-bromo-2-cyclopropyl acetamide (433 mg, 2.43 mmol), cesium carbonate (793 mg, 2.43 mmol) and silver oxide (564 mg, 2.43 mmol) to a solution of (1R,3s,5S)-3-((3S,4R)-1-(5-fluoropyrimidin-2-yl)-3-methoxypiperidin-4-yl)-8-azabicyclo[3.2.1]octane (260 mg, 0.811 mmol) in acetonitrile (4.00 mL), the mixture was stirred for 40 hours at room temperature. The reaction mixture was purified by direct column chromatography (silica gel, 20% methanol/ethyl acetate). The obtained product was dissolved in methanol (10 mL) and supplied onto a Waters Porapak Rxn^{R} CX (two 2 g cartridges). Each solid phase was rinsed with methanol (20 mL), and then each product was eluted with ammonia (2 mol/L methanol solution, 20 mL) and the eluates were combined and concentrated under reduced pressure. The residue was dissolved in methanol and fractionated by supercritical fluid chromatography using a CHIRALPAK^{R} (OD-H/SFC (2 cm × 25 cm) by Daicel (mobile phase: CO₂:methanol (85:15), 120 bar, 40°C, flow rate: 70 mL/min), at 10 mg/500 µL (methanol) per run, obtaining the compound with a retention time of 6.41 minutes (120 mg) as an enantiomeric mixture. The obtained compound was dissolved in methanol and fractionated by supercritical fluid chromatography using a CHIRALPAK^{R} (IG/SFC (2 cm × 25 cm) by Daicel (mobile phase: CO₂:methanol (75:25), 120 bar, 40°C, flow rate: 70 mL/min), at 13 mg/500 µL (methanol) per run, obtaining a compound composed mainly of the subsequently eluting component, with a retention time of 9.11 minutes (75 mg). The obtained compound was dissolved in methanol and fractionated by supercritical fluid chromatography using a CHIRALPAK^{R} (IG/SFC (2 cm × 25 cm) by Daicel (mobile phase: CO₂:methanol (75:25), 120 bar, 40°C, flow rate: 70 mL/min), at 4 mg/100 µL (methanol) per run, obtaining a compound composed mainly of the subsequently eluting component, with a retention time of 7.52 minutes (52 mg). The obtained compound was dissolved in methanol and fractionated by supercritical fluid chromatography using a CHIRALPAK^{R} (IG/SFC (2 cm × 25 cm) by Daicel (mobile phase: CO₂:methanol (75:25), 120 bar, 40°C, flow rate: 70 mL/min), at 7 mg/500 µL (methanol) per run, obtaining the title compound composed mainly of the subsequently eluting component, with a retention time of 7.18 minutes (31.3 mg).

¹H-NMR (400 MHz, CDCl₃) δ(ppm): 0.28-0.37 (m, 1H), 0.45-0.52 (m, 1H), 0.53-0.58 (m, 1H), 0.60-0.68 (m, 1H), 0.78 (dd, J = 8.8,4.3 Hz, 1H), 1.20-1.27 (m, 2H), 1.32-1.45 (m, 2H), 1.46-1.52 (m, 2H), 1.59-1.65 (m, 3H), 1.70-1.80 (m, 2H), 1.81-1.88 (m, 1H), 2.08 (d, J = 9.1 Hz, 1H), 2.68 (dd, J = 14.3,1.1 Hz, 1H), 2.75 (td, J = 12.8,2.9 Hz, 1H), 3.21-3.24 (m, 1H), 3.30 (s, 3H), 3.36 (brs, 1H), 3.86-3.91 (m, 1H), 4.65-4.76 (m, 1H), 5.10 (dt, J = 14.2, 2.4 Hz, 1H), 5.14-5.24 (m, 1H), 6.92-7.02 (m, 1H), 8.14 (s, 2H).
MS (ESI)m/z: 418[M+H]⁺

(Analysis conditions) Chromatography using CHIRALPAK^{R} IA (0.46 cm × 15 cm) by Daicel (mobile phase: ethanol:hexane (20:80), 40°C, flow rate: 1 mL/min, detection: UV (254 nm)).

(Analysis results) The retention time of the title compound was 4.91 minutes and the optical purity was >99% ee.

### Preparation and X-ray crystallographic analysis of (2R)-2-cyclopropyl-2-{(1R.3S,5S)-3-[(3S,4R)-1-(5-fluoropyrimidin-2-yl)-3-methoxypiperidin-4-yl]-8-azabicyclo[3.2.1]octan-8-yl}acetamide single crystals

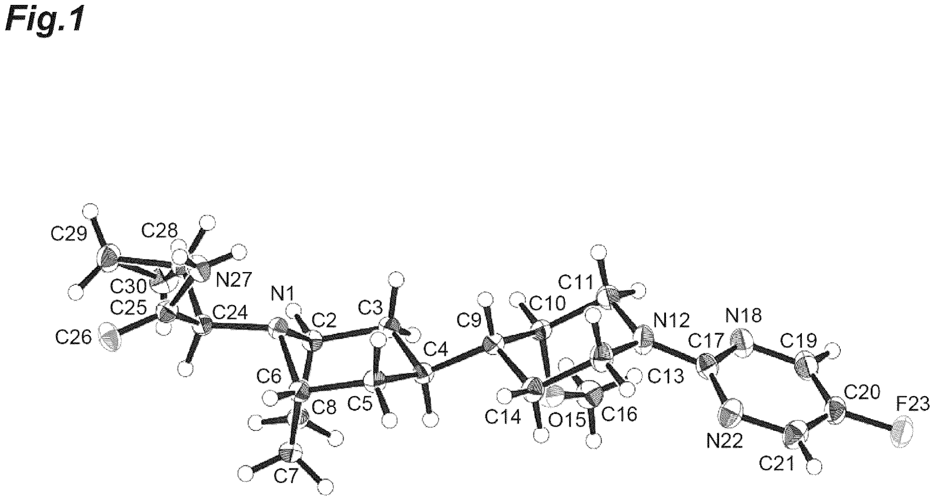

The title compound obtained in Reference Example 6 (2.97 mg) was dissolved in methanol (1 mL). After placing 500 µL of the solution in a vial, the cap was gently closed (solvent evaporation method). After 1 day, a single crystal of the title compound was obtained in the vial. The obtained single crystal was subjected to X-ray crystallographic analysis under the following conditions. The X-ray crystal structure of the title compound is shown in Fig. 1.
Analytical instrument: XtaLAB PRO P200 MM007HF (Rigaku, Japan)
Software: CrysAlisPro (Rigaku Oxford Diffraction)
X-rays: Multi-layer mirror monochromated Cu-Kα (40 kV/30 mA)
Measuring method: Omega axis oscillation method
Camera length: 35 mm
Measuring temperature: -170°C

### Example 1

### Preparation of Form α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl]acetamide

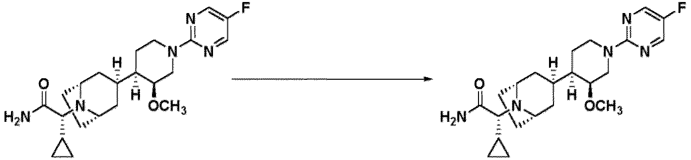

A suspension of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide (1.90 g, content: 92.6%, 4.21 mmol) in 2-propyl alcohol (10 mL) was stirred at 70°C to dissolve the starting material. The obtained solution was stirred at the same temperature for 10 minutes, at room temperature for 4 hours and 35 minutes, and for 1 hour while cooling on ice. The precipitated solid was filtered with a glass filter, rinsed with ice-cooled 2-propyl alcohol (3 mL) and dried under reduced pressure to obtain the title compound (1.46 g). ¹H-NMR of the compound matched the ¹H-NMR of Reference Example 6.
Powder X-ray diffraction peaks (2θ ±0.2°): 10.6°, 11.1°, 12.2°, 12.8°, 15.5°, 21.2°, 23.0°, 24.6°, 26.0°, 35.0°
¹³C-NMR (100 MHz, solid state) δ(ppm): 16.4, 27.7, 49.2, 55.9, 60.9, 67.6, 72.5, 144.4, 149.0, 158.4, 179.1
Raman shift peaks (cm⁻¹): 418.7, 508.1, 778.4, 811.6, 834.5, 842.1, 895.0, 934.9, 1074.8, 1094.2, 1199.4

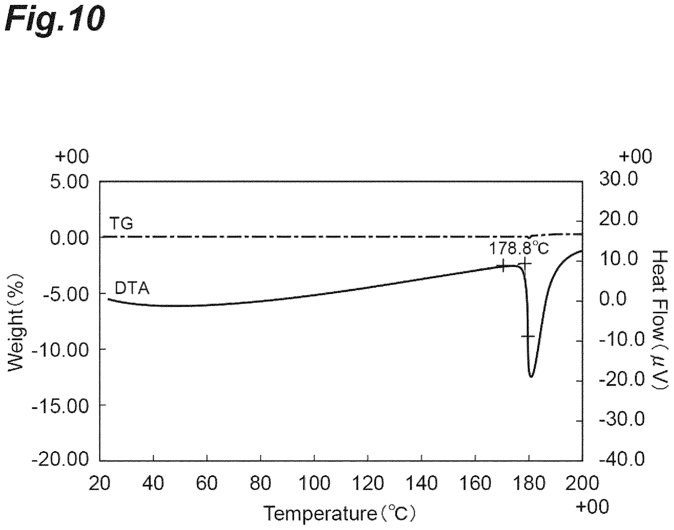

The powder X-ray diffraction pattern of the Form α crystal of compound (I) obtained by this method is shown in Fig. 2, the solid state ¹³C NMR spectrum is shown in Fig. 6, the thermal analysis TG-DTA chart is shown in Fig. 10, and the Raman spectrum is shown in Fig. 14.

### Hygroscopicity test for Form α crystal of compound (I)

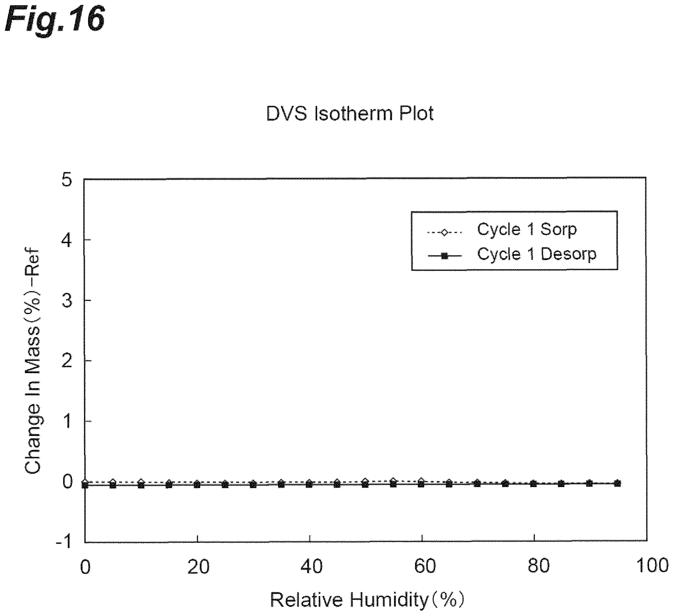

The hygroscopicity of the Form α crystal of compound (I) was evaluated using a dynamic moisture adsorption analyzer. The sample mounting part of the apparatus was kept at 25°C and the relative humidity (RH) was set stepwise in a range of 0% to 95%. The humidity was adjusted by varying the relative flow rates of dry nitrogen at 0% RH and humidified nitrogen at 100% RH. Fig. 16 shows a hygroscopic DVS chart for Form α crystal of compound (I).

### Example 2

### Preparation of Form β crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide

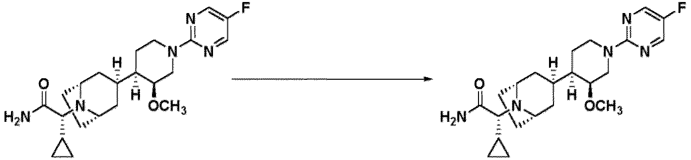

(2R)-2-Cyclopropyl-2-{(1R,3 S,5 S)-3-[(3 S,4R)-1-(5-fluoropyrimidin-2-yl)-3-methoxypiperidin-4-yl]-8-azabicyclo[3.2.1]octan-8-yl}acetamide (1.01 g, 2.40 mmol) was dissolved in 80% ethanol (ethanol:water = 4:1, 30 mL). The resulting solution was concentrated under reduced pressure. The concentrated residue was dried under reduced pressure to obtain the title compound (950 mg). ¹H-NMR of the compound matched the ¹H-NMR of Reference Example 6.
Powder X-ray diffraction peaks (2θ ±0.2°): 4.9°, 9.8°, 11.8°, 14.6°, 15.0°, 16.1°, 19.6°, 20.4°, 20.7°, 23.5°
¹³C-NMR (100 MHz, solid state) δ(ppm): 28.6, 39.1, 56.3, 60.4, 70.6, 74.1, 145.0, 150.5, 159.2, 177.7
Raman shift peaks (cm⁻¹): 716.6, 778.4, 811.6, 837.0, 889.9, 1035.9, 1072.4, 1089.4, 1194.6, 1442.2

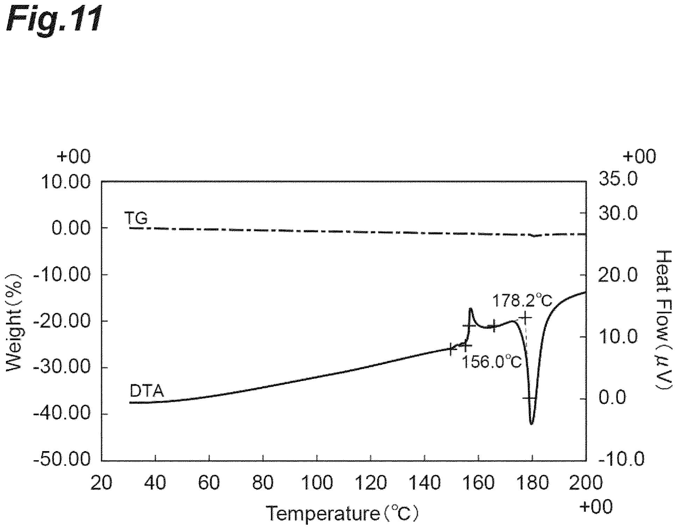

The powder X-ray diffraction pattern of the Form β crystal of compound (I) obtained by this method is shown in Fig. 3, the solid state ¹³C NMR spectrum is shown in Fig. 7, the thermal analysis TG-DTA chart is shown in Fig. 11, and the Raman spectrum is shown in Fig. 15.

### Example 3

### Preparation of crystal of (2R)-2-cyclopropyl-2-{(1R3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate

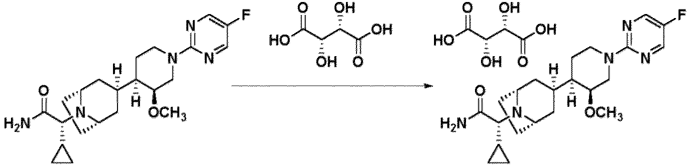

A solution of D-tartaric acid (180 mg, 1.20 mmol) (CAS No. 147-71-7) in 10% methanol/ethyl acetate (5 mL) was added dropwise to a solution of (2R)-2-cyclopropyl-2- { (1R,3 S, 5 S)-3 -[(3 S,4R)-1 -(5-fluoropyrimidin-2-yl)-3 - methoxypiperidin-4-yl]-8-azabicyclo[3.2.1]octan-8-yl}acetamide (500 mg, 1.20 mmol) in 10% methanol/ethyl acetate (5 mL) that had been stirred at 50°C. The obtained mixture was stirred at the same temperature for 20 minutes and then overnight at room temperature. The precipitated solid was filtered with a glass filter, rinsed with ethyl acetate (7 mL) and dried under reduced pressure to obtain the title compound (582 mg).

¹H-NMR (500 MHz, METHANOL-d₄) δ(ppm): 0.53-0.64 (m, 2H), 0.65-0.78 (m, 1H), 0.87-0.99 (m, 1H), 1.14-1.29 (m, 1H), 1.37-1.65 (m, 4H), 1.78-1.97 (m, 6H), 2.01-2.19 (m, 2H), 2.74 (d, J = 14.5 Hz, 1H), 2.76-2.86 (m, 1H), 3.03 (brd, J = 6.9 Hz, 1H), 3.30 (d, J = 1.4 Hz, 3H), 3.40 (brs, 1H), 3.85 (brs, 1H), 4.36 (d, J = 2.1 Hz, 2H), 4.41(brs, 1H), 4.68-4.78 (m, 1H), 5.16 (dd, J = 14.5,1.4 Hz, 1H), 8.20 (d, J = 1.4 Hz, 2H).
Powder X-ray diffraction peaks (2θ ±0.2°): 4.1°, 10.6°, 12.8°, 15.6°, 16.1°, 17.7°, 19.6°, 20.0°, 23.3°, 32.3°
¹³C-NMR (100 MHz, solid state) δ(ppm): 13.2, 26.6, 42.3, 58.6, 60.6, 62.0, 71.6, 73.8, 143.9, 171.2, 174.1, 179.3

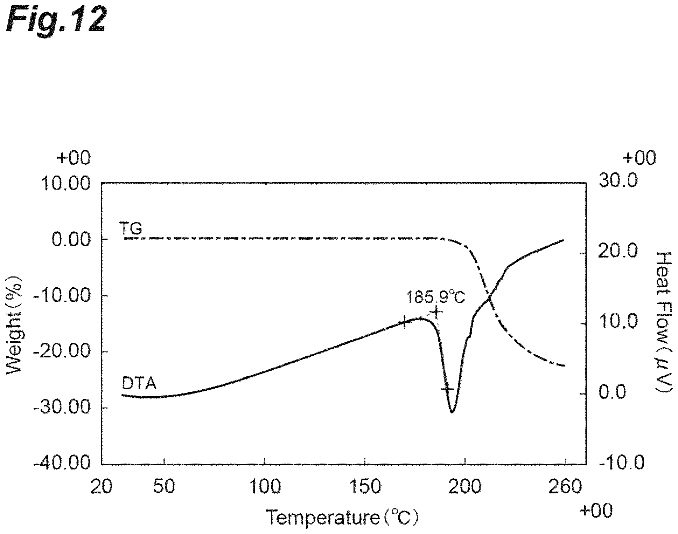

The powder X-ray diffraction pattern of the crystal of compound (I) mono D-tartrate obtained by this method is shown in Fig. 4, the solid state ¹³C NMR spectrum is shown in Fig. 8, and the thermal analysis TG-DTA chart is shown in Fig. 12.

### Example 4

### Preparation of crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate

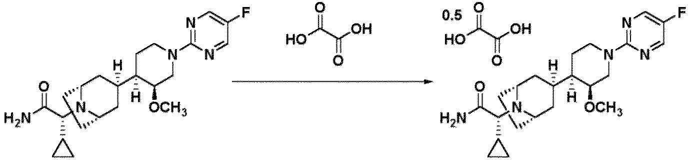

A solution of oxalic acid(2.91 g, 32.3 mmol) (CAS No. 144-62-7) in methanol (14 mL) was added dropwise over 10 minutes to a solution of (2R)-2-cyclopropyl-2-f (1R,3S,SS)-3-[(3S,4R)-1-(5-fluoropyrimidin-2-yl)-3-methoxypiperidin-4-yl]-8-azabicyclo[3.2.1]octan-8-yl}acetamide (38.6 g, content: 70%, 64.8 mmol) in ethyl acetate (680 mL) that had been stirred at 50°C. The obtained mixture was cooled to room temperature and stirred for 5 days. The precipitated solid was filtered, rinsed with ethyl acetate (100 mL) and dried under reduced pressure to obtain the title compound (30.7 g).

¹H-NMR (400 MHz, METHANOL-d₄) δ(ppm): 0.49-0.61 (m, 2H), 0.61-0.71 (m, 1H), 0.85-0.96 (m, 1H), 1.11-1.21 (m, 1H), 1.36-1.63 (m, 4H), 1.69-1.93 (m, 6H), 2.03-2.11 (m, 2H), 2.76 (dd, J = 14.3,1.1 Hz, 1H), 2.79-2.92 (m, 2H), 3.32 (s, 3H), 3.43(brs, 1H), 3.71(brs, 1H), 4.28(brs, 1H), 4.68-4.81 (m, 1H), 5.17 (dt, J = 14.4,2.3 Hz, 1H), 8.22 (s, 2H).
Powder X-ray diffraction peaks (2θ ±0.2°): 5.0°, 5.8°, 7.2°, 8.6°, 9.7°, 10.0°, 12.3°, 13.2°, 14.3°, 14.9°
¹³C-NMR (100 MHz, solid state) δ(ppm): 27.3, 44.1, 55.2, 62.4, 67.5, 74.4, 79.7, 149.9, 159.3, 172.5

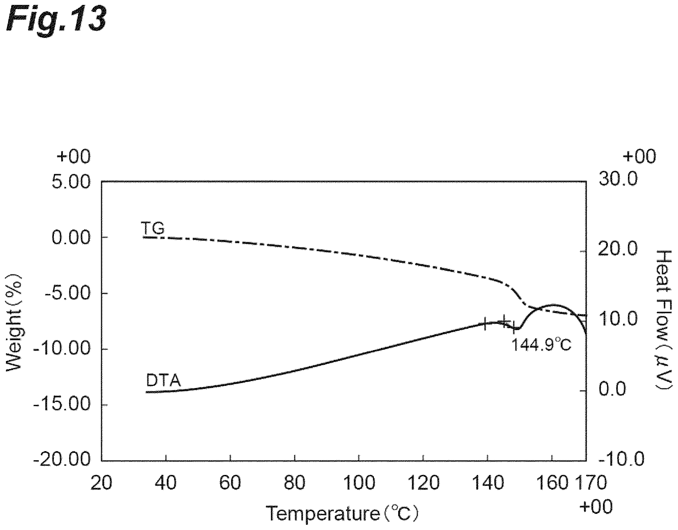

The powder X-ray diffraction pattern of the crystal of compound (I) hemioxalate obtained by this method is shown in Fig. 5, the solid state ¹³C NMR spectrum is shown in Fig. 9, and the thermal analysis TG-DTA chart is shown in Fig. 13.

### <Pharmacological Test Examples>

### Test Example 1-1: Evaluation of activating activity for OX1R and OX2R

Human Embryonic Kidney cells 293 (HEK293) cells with forced expression of hOXIR and hOX2R were seeded in a 384-well microplate (Greiner) at 10,000 per well and cultured for 24 hours in high-glucose-DMEM (FujiFilm Corp.-Wako Pure Chemical Industries, Ltd.) containing added 10% FBS (Thermo Scientific) and 1% Penicillin-Streptomycin (FujiFilm Corp.-Wako Pure Chemical Industries, Ltd.). After removing the medium, there was added 40 µL of assay buffer (20 mM HEPES (Sigma-Aldrich Japan, KK.), Hank's Balanced Salt Solution (Gibco), 0.1% BSA (Sigma-Aldrich Japan, KK.), 0.1% Pluronic F-127 (Biotium, Inc.)) containing Calcium 4 dye (Molecular Device Corporation) and 2.5 mM probenecid (Sigma-Aldrich Japan, KK.), and the mixture was incubated for 60 minutes. After further adding 20 µL of assay buffer, 20 µL of assay buffer containing the test compound was added and reaction was initiated. Change in intracellular calcium ion concentration by the reaction was measured based on the fluorescence intensity ratio in terms of the fluorescence value with dual wavelength excitation at 480 nm and 540 nm, using FDSS7000 (Hamamatsu Photonics, K.K.). The test compound was dissolved in DMSO to 10 mM, and diluted with assay buffer to a final concentration of from 3 × 10⁻¹⁰ M to 1 × 10⁻⁷ M (DMSO final concentration of 0.1%). The 50% activation concentration (EC50 value) was determined from the fluorescence value with addition of the test compound at different concentrations, with the fluorescence value of the well with added compound-free buffer as 0%, and the fluorescence value of the well with 10 nM OX-A (Peptide Research Lab) as 100%. The activating activity value of compound (I) is shown in Table 1.

**[Table 1]**

| | hOX1R EC50 (nM) | hOX2R EC50 (nM) |
|---|---|---|
| Compound (I) | >100 | 0.99 |

Test Example 1-2: Evaluation of activating activity for OX1R and OX2R Human Embryonic Kidney cells 293 (HEK293) cells with forced expression of hOXIR and hOX2R were seeded in a 384-well microplate (Greiner) at 10,000 per well and cultured for 1 day in high-glucose-DMEM (FujiFilm Corp.-Wako Pure Chemical Industries, Ltd.) containing added 10% FBS (Thermo Scientific) and 1% Penicillin-Streptomycin (FujiFilm Corp.-Wako Pure Chemical Industries, Ltd.). After removing the medium, there was added 30 µL of assay buffer (20 mM HEPES (Sigma-Aldrich Japan, KK.), Hank's balanced salt solution (Gibco), 0.1% BSA (Sigma-Aldrich Japan, KK.), 0.1% Pluronic F-127 (Biotium, Inc.)) containing Calcium 4 dye (Molecular Device Corporation) and 2.5 mM probenecid (Sigma-Aldrich Japan, KK.), and the mixture was incubated for 60 minutes. A 30 µL portion of assay buffer containing the test compound was added and reaction was initiated. Change in intracellular calcium ion concentration by the reaction was measured based on the fluorescence intensity ratio in terms of the fluorescence value with dual wavelength excitation at 480 nm and 540 nm, using FDSS7000 (Hamamatsu Photonics, K.K.). The test compound was dissolved in DMSO to 10 mM, and diluted with assay buffer to a final concentration of from 3 × 10⁻¹¹ M to 1 × 10⁻⁵ M (DMSO final concentration of 0.1%). The 50% activation concentration

(EC50 value) was determined from the fluorescence value with addition of the test compound at different concentrations, with the fluorescence value of the well with added compound-free buffer as 0%, and the fluorescence value of the well with 10 nM OX-A (Peptide Research Lab) as 100%. The activating activity value of compound (I) is shown in Table 2.

**[Table 2]**

| | hOX1R EC50 (nM) | hOX2R EC50 (nM) |
|---|---|---|
| Compound (I) | 4700 | 2.3 |

Test Example 2: Increase in spontaneous movement Increased movement in mice is an indicator of increased alertness time, higher body temperature and augmented cardiovascular parameters, as well as heightened alertness. In this test example, the alertness effect was evaluated by measuring spontaneous movement of the mice. Male C57BL/6NCrl mice (18-19 weeks old, Charles River Laboratories, Japan Inc., 4 mice in each group) were used for the experiment. Spontaneous movement was measured by using a movement measuring device (VersaMax Open Field, AccuScan Instruments, Inc.), irradiating infrared rays from the side sections of the measuring cage, and quantifying the number of times that the mice passed through the irradiated rays. After placing the mice in the measuring cage and conditioning for 3 hours, compound (I) was orally administered (10 mg/kg). Spontaneous movement was measured 2 hours after administration. The mice in the compound (I)-administered group were administered a solution of compound (I) dissolved in 0.1 mol/L hydrochloric acid containing 5% (v/v) DMSO and 5% (v/v) Kolliphor^{R} EL. For the control group, solvent alone without compound (I) was administered to the mice.

The results are shown in Table 3. Spontaneous movement was represented as a percentage for the compound (I)-administered group, with spontaneous movement in the control group as 100%.

**[Table 3]**

| Test compound | Spontaneous movement (% of Control) |
|---|---|
| Solvent | 100 |
| Compound (I) | 865 |

As clearly seen from Table 3, compound (I) augmented spontaneous movement in the mice. In other words, compound (I) was shown to have an alertness effect.

### Test Example 3: Alertness effect by dark period oral administration of compound (I) to wild type mice

The experimental animals used were C57BL/6 line wild type (WT) male mice. Electroencephalogram and electromyogram measuring electrodes were surgically embedded into 13-week-old mice under isoflurane anesthesia. After surgery, and following conditioning to the illumination cycle and experimentation procedure, electroencephalogram and electromyogram measurement were carried out and mice with proper electroencephalogram and electromyogram recordings were supplied for the experiment. The solvent (0 mg/kg) or a solution of compound (I) dissolved in the solvent (1, 3 or 10 mg/kg) was orally administered 30 to 15 minutes before lighting was turned off. The solvent used was a 0.1 mol/L hydrochloric acid solution containing 5% (v/v) DMSO and 5% (v/v) Kolliphor^{R} EL. The electroencephalogram and electromyogram were recorded for about 24 hours, from 1 hour before lighting was turned off. The mice were repeatedly used, with a washout period of 2 days or longer. The electroencephalogram and electromyogram data obtained for each mouse were used to assess the sleep stage every epoch (10 seconds), using sleep analysis software (SleepSign: Kissei Comtec Co., Ltd.). The time (sleep latency) until initial sleep was exhibited after lighting out (sleeping for 8 or more epochs beginning after non-REM sleep) was measured for each mouse. Using 16 mice in each administered group, the sleep latency in a solvent-administered group (control group) and a compound (I)-administered group were compared by a Dunnet-type multiple comparison test following survival time analysis in consideration of the number of experiments and use of the same individuals, with a significance level of 5% at both ends for each.

The sleep latencies of the mice administered the solvent and compound (I) at 1, 3 and 10 mg/kg were 0.23 hour, 0.28 hour, 0.44 hour and 2.07 hours, respectively. In the compound (I)-administered groups with 3 or 10 mg/kg, the sleep latency increased significantly with respect to the solvent-administered group. Specifically, when compound (I) was orally administered during the light period (ZT12), which was the beginning of the active period for the mice, sleep latency was found to be lengthened in a dose-dependent manner.

### Test Example 4: Alertness effect and cataplexy-like behavior inhibiting effect by dark period oral administration of compound (I) to orexin-knockout mice (orexin/ataxin 3 mice)

The experimental animals used were orexin/ataxin 3 mice (orexin/ataxin-3 Tg/+ (hereunder referred to as "Tg mice"), Hara et al., Neuron, 30, 345-54, 2001) with a C57BL/6 genetic background, with wild type mice (hereunder referred to as "WT mice") of the same litter as the control. Electroencephalogram and electromyogram measuring electrodes were surgically embedded into 12-week-old mice (±2 weeks) under isoflurane anesthesia. After surgery, and following conditioning to the illumination cycle and experimentation procedure, electroencephalogram and electromyogram measurement were carried out and mice with proper electroencephalogram and electromyogram recording were supplied for the experiment. The solvent (0 mg/kg) or a specimen (solution of compound (I) dissolved in the solvent (0.3, 1 or 3 mg/kg)) was orally administered 30 to 15 minutes before lighting was turned off. The solvent used was a 0.1 mol/L hydrochloric acid solution containing 5% (v/v) DMSO and 5% (v/v) Kolliphor^{R} EL. The electroencephalogram and electromyogram were recorded for about 24 hours, from 1 hour before lighting was turned off. The mice were repeatedly used, with a washout period of 2 days or longer. The electroencephalogram and electromyogram data obtained for each mouse were used to assess the sleep stage every epoch (10 seconds), using sleep analysis software (SleepSign: Kissei Comtec Co., Ltd.), up to a maximum of 4 hours. For the purpose of this experiment, cataplexy-like symptoms were defined as REM sleep appearing immediately after wakefulness (direct transitions from wake to REM sleep (DREM)) for a contiguous period of 4 epochs or longer. DREM in mice is an analog of cataplexy (Exp Neurol. 2009; 217:46-54). The time until initial sleep appeared after lighting out for each mouse (sleep for contiguous 8 epochs or longer, excluding DREM) (sleep latency) and the time until initial DREM appeared (DREM latency) were measured. The numbers of mice were 8 in the solvent-administered group and 14 in the compound (I)-administered group. The sleep latency in a disease control group and a compound (I)-administered group were compared by a Dunnet-type multiple comparison test following survival time analysis in consideration of the number of experiments and use of the same individuals, with a significance level of 5% at both ends for each. The DREM latency in the normal control group and disease control group were compared by survival time analysis in consideration of the number of experiments and use of the same individuals. Cases with significance in the statistical test for the disease control group and the compound (I)-administered group were compared by a Dunnet-type multiple comparison test following survival time analysis in consideration of the number of experiments and use of the same individuals, with a significance level of 5% at both ends for each.

The sleep latencies for Tg mice administered the solvent and the Example 1 compound at 0.3, 1 and 3 mg/kg were 0.21 hour, 0.31 hour, 0.64 hour and 2.42 hours, respectively, indicating significant increase in sleep latency in the compound (I)-administered groups with 1 and 3 mg/kg. Specifically, when compound (I) was orally administered during the light period (ZT12), which was the beginning of the active period for the mice, sleep latency was found to be lengthened in a dose-dependent manner from 1 mg/kg in the orexin-knockout mice.

The DREM latency with administration of medium in the WT mice was 4.00 hours. However, the DREM latencies with administration of solvent and administration of compound (I) at 0.3, 1 and 3 mg/kg in the Tg mice were 1.16 hours, 1.50 hours, 2.26 hours and 4.00 hours, respectively, confirming that DREM latency was increased significantly and in a dose-dependent manner in the compound (I)-administered groups at 0.3, 1 and 3 mg/kg, compared to the solvent-administered group. In other words, cataplexy-like symptoms (DREM) exhibited by orexin-knockout mice were inhibited by administration of compound (I), in a dose-dependent manner.

## Claims

1. (2R)-2-cyclopropyl-2-{(1R,3S, 5 S)-3-[(3 S,4R)-1-(5-fluoropyrimidin-2-yl)-3-methoxypiperidin-4-yl]-8-azabicyclo[3.2.1]octan-8-yl}acetamide mono D-tartrate represented by formula (II): 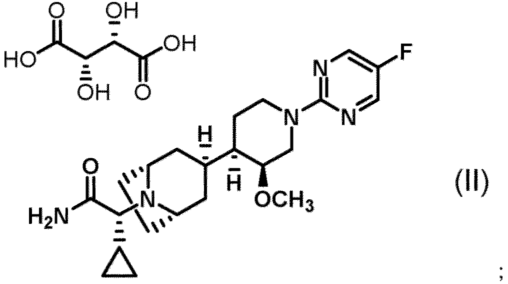 or
(2R)-2-cyclopropyl-2-{(1R,3S, 5 S)-3-[(3 S,4R)-1-(5-fluoropyrimidin-2-yl)-3-methoxypiperidin-4-yl]-8-azabicyclo[3.2.1]octan-8-yl}acetamide hemioxalate represented by formula (III):

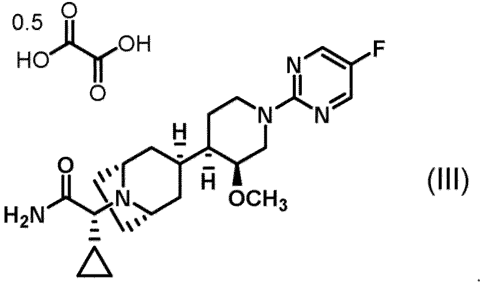

2. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate represented by formula (II): 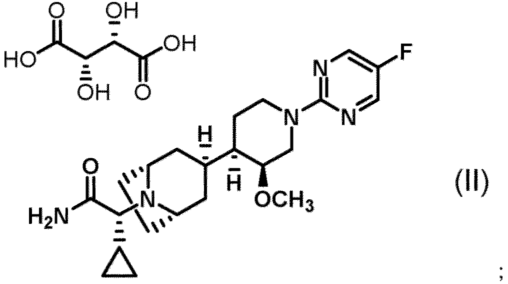 or
a crystal of (2R)-2-cyclopropyl-2-{(1R,3S,SS)-3-[(3S,4R)-1-(5-fluoropyrimidin-2-yl)-3-methoxypiperidin-4-yl]-8-azabicyclo[3.2.1]octan-8-yl}acetamide hemioxalate represented by formula (III):

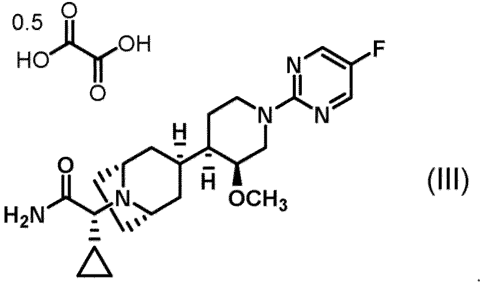

3. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 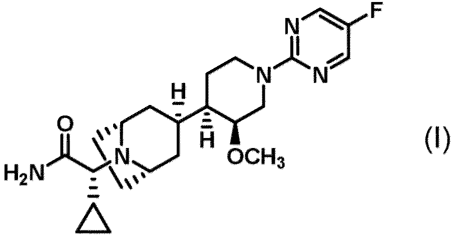 having one or more diffraction peaks selected from the group consisting of diffraction angles (2θ ±0.2°) of 12.2°, 12.8°, 15.5°, 21.2° and 24.6° in a powder X-ray diffraction.

4. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 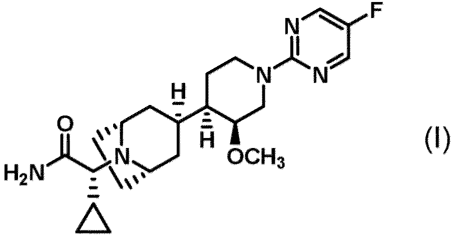 having a diffraction peak at a diffraction angle (2θ ±0.2°) of 24.6° in a powder X-ray diffraction.

5. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 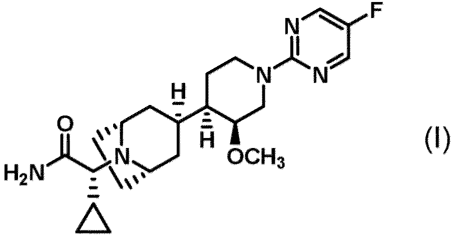 having diffraction peaks at diffraction angles (2θ ±0.2°) of 12.2°, 15.5° and 24.6° in a powder X-ray diffraction.

6. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 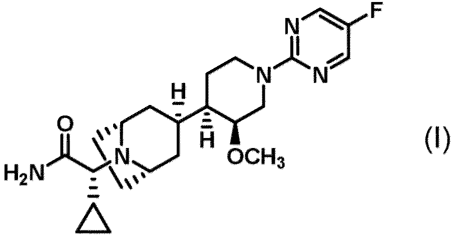 having a powder X-ray diffraction pattern substantially the same as the powder X-ray diffraction pattern shown in Fig. 2.

7. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 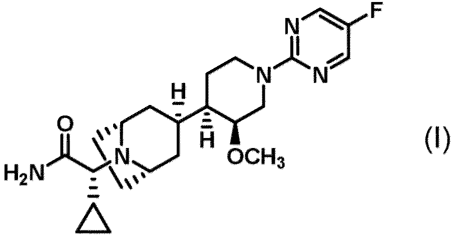 having a peak at a chemical shift (δ ±0.5 ppm) of 72.5 ppm in a solid state ¹³C NMR spectrum.

8. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 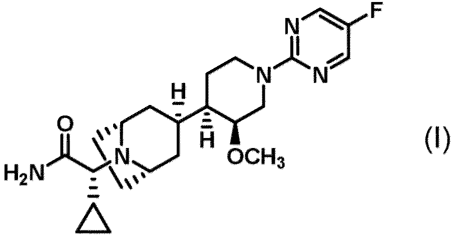 having peaks at chemical shifts (δ ±0.5 ppm) of 49.2 ppm, 67.6 ppm and 72.5 ppm in a solid state ¹³C NMR spectrum.

9. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 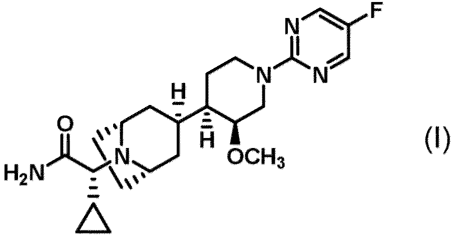 having a solid state ¹³C NMR spectrum substantially the same as the solid state ¹³C NMR spectrum shown in Fig. 6.

10. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 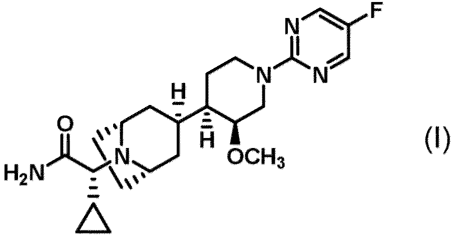 having a Raman shift peak (±2 cm⁻¹) at 1199.4 cm⁻¹ in Raman spectrophotometry.

11. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 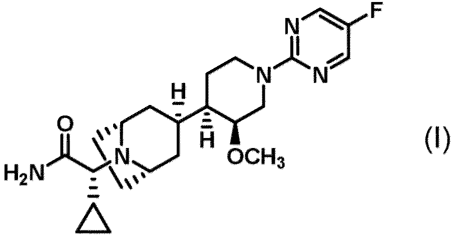 having Raman shift peaks (±2 cm⁻¹) at 842.1 cm⁻¹, 895.0 cm⁻¹ and 1199.4 cm⁻¹ in Raman spectrophotometry.

12. AForm α crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide represented by formula (I): 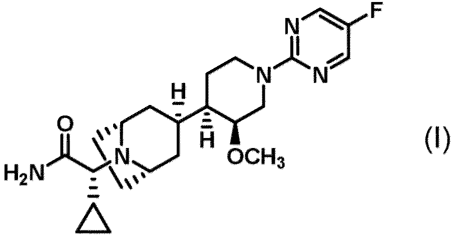 having a Raman spectrum substantially the same as the Raman spectrum shown in Fig. 14.

13. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 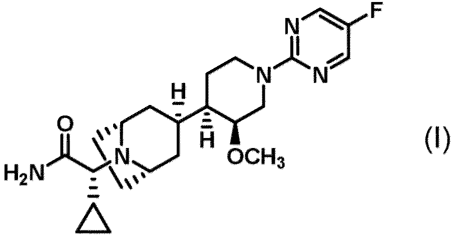 having one or more diffraction peaks selected from the group consisting of diffraction angles (2θ ±0.2°) of 4.9°, 9.8°, 11.8°, 14.6° and 16.1° in a powder X-ray diffraction.

14. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 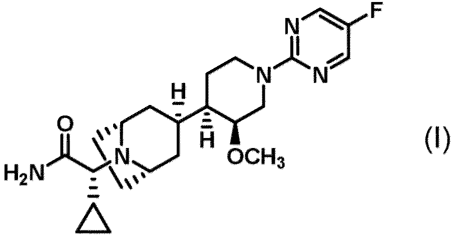 having a diffraction peak at a diffraction angle (2θ ±9.2°) of 14.6° in a powder X-ray diffraction.

15. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 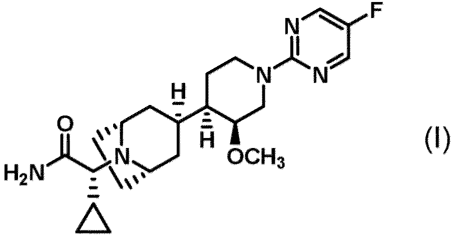 having diffraction peaks at diffraction angles (2θ ±0.2°) of 11.8°, 14.6° and 16.1° in a powder X-ray diffraction.

16. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 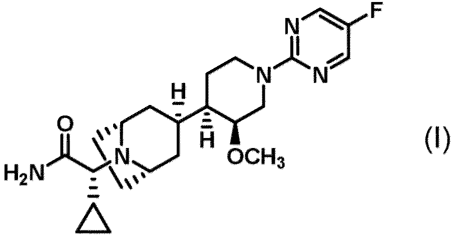 having a powder X-ray diffraction pattern substantially the same as the powder X-ray diffraction pattern shown in Fig. 3.

17. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 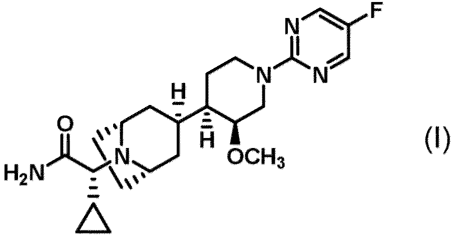 having a peak at a chemical shift (δ ±0.5 ppm) of 74.1 ppm in a solid state ¹³C NMR spectrum.

18. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 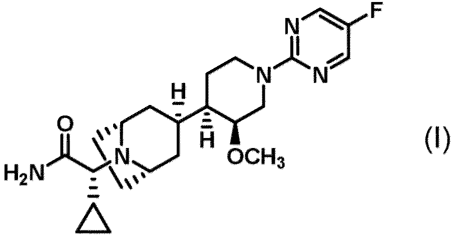 having peaks at chemical shifts (δ ±0.5 ppm) of 74.1 ppm, 159.2 ppm and 177.7 ppm in a solid state ¹³C NMR spectrum.

19. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 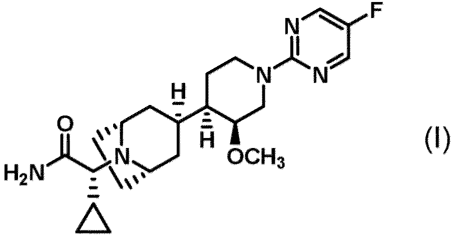 having a solid state ¹³C NMR spectrum substantially the same as the solid state ¹³C NMR spectrum shown in Fig. 7.

20. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 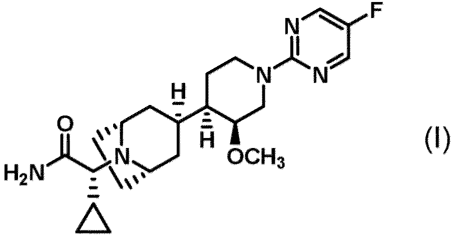 having a Raman shift peak (±2 cm⁻¹) at 1089.4 cm⁻¹ in Raman spectrophotometry.

21. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 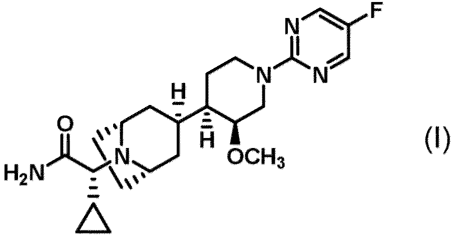 having Raman shift peaks (±2 cm⁻¹) at 889.9 cm⁻¹, 1089.4 cm⁻¹ and 1194.6 cm⁻¹ in Raman spectrophotometry.

22. AForm β crystal of (2R)-2-cyclopropyl-2-f(1R,3S,SS)-3-[(3S,4R)-1-(5-fluoropyrimidin-2-yl)-3-methoxypiperidin-4-yl]-8-azabicyclo[3.2.1]octan-8-yl}acetamide represented by formula (I): 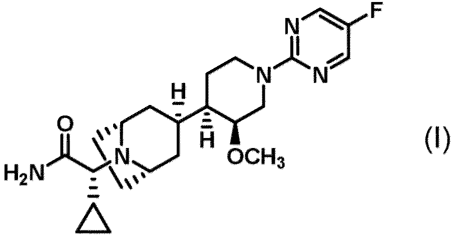 having a Raman spectrum substantially the same as the Raman spectrum shown in Fig. 15.

23. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate represented by formula (II): 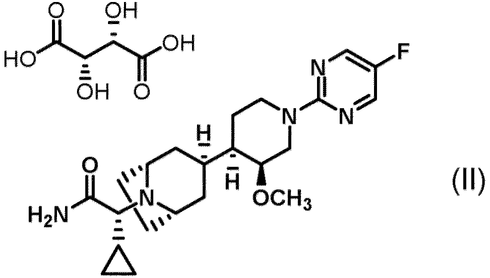 having one or more diffraction peaks selected from the group consisting of diffraction angles (2θ ±0.2°) of 4.1°, 12.8°, 16.1°, 20.0° and 23.3° in a powder X-ray diffraction.

24. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate represented by formula (II): 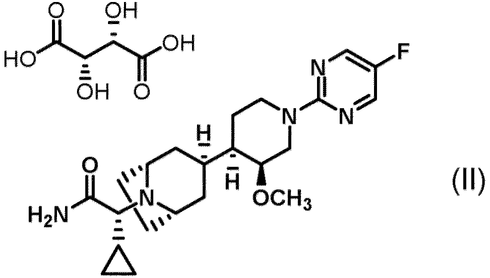 having a diffraction peak at a diffraction angle (2θ ±9.2°) of 4.1° in a powder X-ray diffraction.

25. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate represented by formula (II): 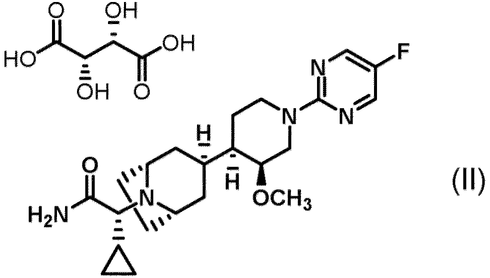 having diffraction peaks at diffraction angles (2θ ±0.2°) of 4.1°, 12.8° and 23.3° in a powder X-ray diffraction.

26. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate represented by formula (II): 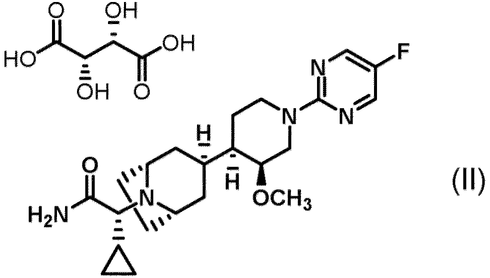 having a powder X-ray diffraction pattern substantially the same as the powder X-ray diffraction pattern shown in Fig. 4.

27. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate represented by formula (II): 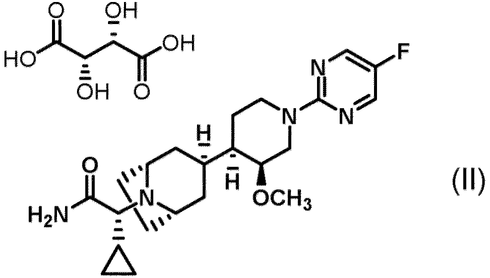 having a peak at a chemical shift (δ ±0.5 ppm) of 73.8 ppm in a solid state ¹³C NMR spectrum.

28. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate represented by formula (II): 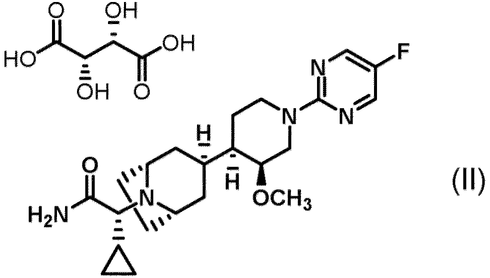 having peaks at chemical shifts (δ ±0.5 ppm) of 71.6 ppm, 73.8 ppm and 179.3 ppm in a solid state ¹³C NMR spectrum.

29. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide mono D-tartrate represented by formula (II): 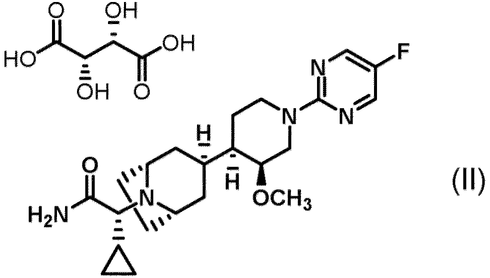 having a solid state ¹³C NMR spectrum substantially the same as the solid state ¹³C NMR spectrum shown in Fig. 8.

30. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate represented by formula (III): 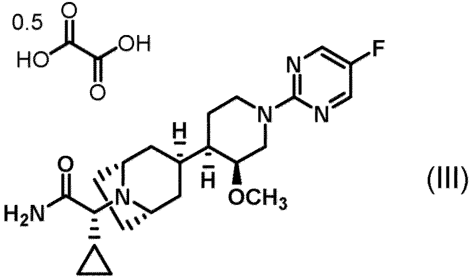 having one or more diffraction peaks selected from the group consisting of diffraction angles (2θ ±0.2°) of 5.0°, 5.8°, 10.0°, 13.2° and 14.3° in a powder X-ray diffraction.

31. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate represented by formula (III): 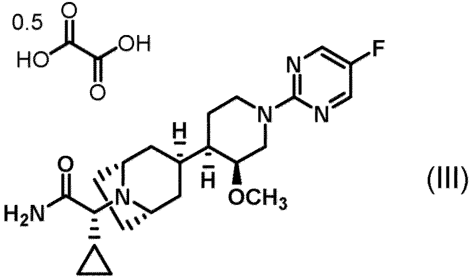 having a diffraction peak at a diffraction angle (2θ ±0.2°) of 10.0° in a powder X-ray diffraction.

32. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate represented by formula (III): 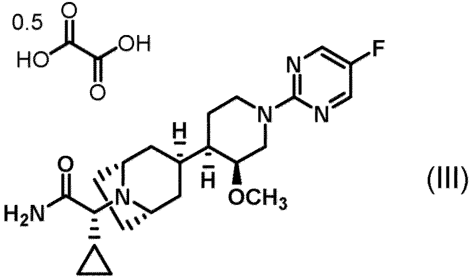 having diffraction peaks at diffraction angles (2θ ±0.2°) of 5.8°, 10.0° and 14.3° in a powder X-ray diffraction.

33. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate represented by formula (III): 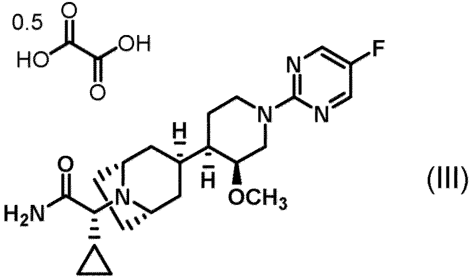 having a powder X-ray diffraction pattern substantially the same as the powder X-ray diffraction pattern shown in Fig. 5.

34. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate represented by formula (III): 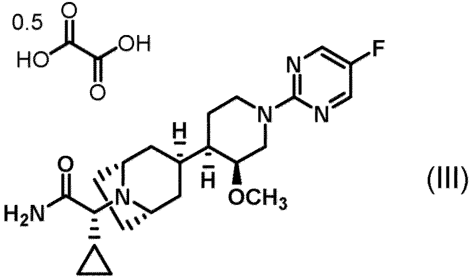 having a peak at a chemical shift (δ ±0.5 ppm) of 44.1 ppm in a solid state ¹³C NMR spectrum.

35. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate represented by formula (III): 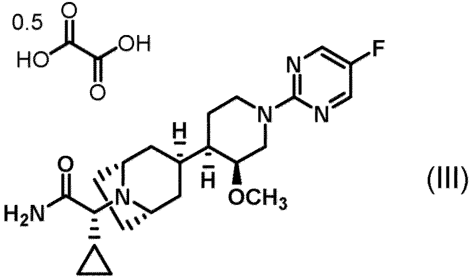 having peaks at chemical shifts (δ ±0.5 ppm) of 27.3 ppm, 44.1 ppm and 62.4 ppm in a solid state ¹³C NMR spectrum.

36. A crystal of (2R)-2-cyclopropyl-2-{(1R,3S,5S)-3-[(3S,4R)-1-(5-fluoropyrimidin-2-yl)-3-methoxypiperidin-4-yl]-8-azabicyclo[3.2.1]octan-8-yl}acetamide hemioxalate represented by formula (III): 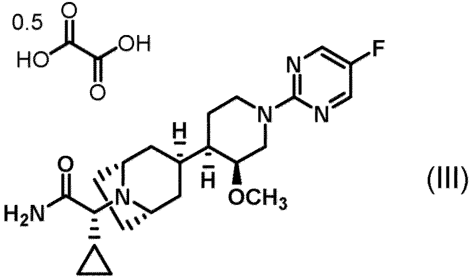 having a solid state ¹³C NMR spectrum substantially the same as the solid state ¹³C NMR spectrum shown in Fig. 9.

37. A pharmaceutical composition comprising the salt according to claim 1.

38. A pharmaceutical composition comprising the crystal according to any one of claims 2 to 36.
